(19) Europäisches Patentamt / European Patent Office / Office européen des brevets

(11) **EP 4 599 843 A1**

(12) **EUROPEAN PATENT APPLICATION**

(43) Date of publication:
**13.08.2025 Bulletin 2025/33**

(21) Application number: **24382133.7**

(22) Date of filing: **09.02.2024**

(51) International Patent Classification (IPC):
**A61K 39/00** (2006.01)

(52) Cooperative Patent Classification (CPC):
**A61K 40/11; A61K 40/30; A61K 40/31;
A61K 40/421; A61K 40/4215; A61K 40/4224;**
A61K 2239/28; A61K 2239/48

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicant: **Fundació Institut de recerca de
l'Hospital de la
Santa Creu i Sant Pau
08041 Barcelona (ES)**

(72) Inventors:
• **BRIONES MEIJIDE, Javier
08041 Barcelona (ES)**

• **ESCRIBÀ GARCÍA, Laura
08041 Barcelona (ES)**
• **ESCUDERO LÓPEZ, Eva
08041 Barcelona (ES)**
• **ÁLVAREZ FERNÁNDEZ, Carmen
08041 Barcelona (ES)**

(74) Representative: **Elion IP, S.L.
Paseo Castellana, 150-4 dcha
28046 Madrid (ES)**

Remarks:
•The complete document including Reference
Table(s) and the Sequence Listing(s) can be
downloaded from the EPO website
•Claim 16 is deemed to be abandoned due to non-
payment of the claims fee (Rule 45(3) EPC).

(54) **CAR AND MODIFIED CD200R COMBINATION**

(57) The present disclosure relates to a combination of a chimeric antigen receptor (CAR) and a modified CD200 receptor (CD200R). More in particular, a combination of a CAR targeting an antigen highly expressed in cancers which also typically express CD200, such as Hodgkin lymphoma, with a modified CD200R has been found useful in the treatment of such cancers. The present disclosure further relates to polynucleic acids, vectors, immune cells, pharmaceutical compositions encoding or comprising said combination, the same for use in the treatment of cancer and methods of preparation of said immune cells.

FIG. 2

EP 4 599 843 A1

## Description

### TECHNICAL FIELD

The present disclosure relates to the fields of molecular biology, cell biology, and cancer biology. In particular, the present disclosure relates to a combination of a chimeric antigen receptor (CAR) and a modified CD200 receptor (CD200R). More in particular, a combination of a CAR targeting an antigen highly expressed in cancers which also typically express CD200, such as Hodgkin lymphoma, with a modified CD200R has been found useful in the treatment of such cancers. The present disclosure further relates to polynucleic acids, vectors, immune cells, pharmaceutical compositions encoding or comprising said combination, the same for use in the treatment of cancer and methods of preparation of said immune cells.

### BACKGROUND

CAR T-cell technology is an active field of research in the treatment of cancers, whereby T-cells taken from a patient's blood are modified for them to express so called chimeric antigen receptors (CARs), which are proteins designed to target specific antigens expressed in cancer cells. Thereby, once the modified T-cells are reintroduced to the patient's body, the CARs help the T-cells attach to specific cancer cells. Different cancers have different antigens. Accordingly, to treat a specific cancer a CAR may be tailored for its specific cancer's antigen. For example, one of the existing CAR T-cell technologies target the antigen CD19 (Cluster of Differentiation 19), which is expressed in certain kinds of leukemia or lymphoma. To treat these cancers, T-cells have been modified with CARs designed to attach to the CD19 antigen.

Given the success of CAR T-cells directed to CD19, new targets are being developed and tested, since not all lymphomas express CD19. Other CARs have been designed to target other types of antigens highly expressed in cancer cells and, in particular, in leukemia or lymphoma such as Hodgkin lymphoma (HL). CD30 (Cluster of Differentiation 30), a member of the tumor necrosis factor receptor family, which is highly expressed by HL malignant cells, is a promising target as it is universally expressed in virtually all classical Hodgkin lymphomas, while its expression is highly restricted in healthy cells, making this receptor a very convenient target for Hodgkin and other CD30+ lymphomas such as primary mediastinal non-Hodgkin lymphoma. For instance, the international application published as WO 2020/135559 describes CD30-binding moieties, chimeric antigen receptors (CARs) comprising such CD30-binding moieties (also referred to herein as CD30-CAR or CAR30), genetically engineered immune cells expressing such CD30-CARs, and uses thereof in treating CD30-expressing tumors or cancers. Two reported clinical trials using CD30-CAR T-cells against HL by Wang *et al.* 2017 (Wang et al. Autologous T-cells expressing CD30 chimeric antigen receptors for relapsed or refractory hodgkin lymphoma: an open-label phase I trial. Clin Cancer Res 2017; 23: 1156-1166) and Ramos *et al.* 2020 (Ramos et al. Anti-CD30 CAR-T-cell therapy in relapsed and refractory Hodgkin lymphoma. J Clin Oncol 2020; 38: 3794-3804), have demonstrated that CD30-CAR T-cell infusion is well tolerated, with no relevant toxicities. However, although clinical results in those heavily treated patients are promising, responses are modest, and, in contrast to what is seen in patients treated with a CAR directed to CD19 (CD19-CAR or CAR19), very few patients achieve long-term remission.

Accordingly, there is a need of improved CAR T-cell therapies targeting antigens highly expressed in cancerous tumors, in particular hematological tumors, and more in particular in Hodgkin lymphoma, and primary mediastinal non-Hodgkin lymphoma. For instance, antigens of interest may include an antigen selected from a CD30 antigen, a CD15 antigen, and a CD123 antigen, all of which appear to be highly expressed in such aforementioned types of cancerous tumors.

It has now been found that CARs targeting such antigens can be combined with modified CD200 receptors (CD200Rs) to provide CAR T-cells for improved CAR T-cell therapies, particularly since antigens such as CD30 antigen, a CD15 antigen, and a CD123 antigen have been found to often be co-expressed with CD200 in such cancerous cells.

CD200 (Cluster of Differentiation 200), also known as OX-2 membrane glycoprotein, is a human protein that acts as a negative regulator of T-cell function that binds CD200 receptor (CD200R). Expression of CD200 is commonly increased in leukemia and other malignancies and is associated with poor prognosis in leukemia and lymphoma patients, including Hodgkin lymphoma, and primary mediastinal non-Hodgkin lymphoma. Oda *et al.* 2017 described a CD200R-CD28 fusion protein able to appropriate the inhibitory signal of CD200 and redirect the inhibitory effects to enhance T-cell function, for its ultimate use in the therapy of acute myeloid leukemia (Oda et al. A CD200R-CD28 fusion protein appropriates an inhibitory signal to enhance T-cell function and therapy of murine leukemia. Blood 2017; vol. 130 num. 22: 2410-2419, and international patent applications published as WO 2016/141357 and WO 2018/170475).

### SUMMARY

As indicated above, it has now been found that CARs targeting antigens selected from a CD30 antigen, a CD15 antigen, and a CD123 antigen can be combined with modified CD200 receptors (CD200Rs) to provide CAR T-cells for improved CAR T-cell therapies. In particular, a combination of CD30-CAR with modified CD200R has been found to improve *in vitro* and *in vivo* results against cancer. As it will be discussed in more detail below.

Accordingly, the present invention is directed to a protein combination comprising a) a chimeric antigen receptor (CAR) having an antigen binding domain that specifically binds an antigen selected from a CD30 antigen, a CD15 antigen a CD123 antigen, and a and b) a modified CD200 receptor (CD200R); to a polynucleic acid or a polynucleic acid combination comprising a first polynucleotide encoding the CAR (a) and a second polynucleotide encoding the modified CD200R (b) of said protein combination; a vector or a vector combination respectively comprising said polynucleic acid or polynucleic acid combination; an immune cell comprising said protein combination, polynucleic acid or polynucleic acid combination and/or vector or vector combination; a method of manufacturing said an immune cell; a pharmaceutical composition comprising said protein combination, polynucleic acid or polynucleic acid combination, vector or vector combination and/or said immune cell; and said protein combination, polynucleic acid or polynucleic acid combination, vector or vector combination; immune cell or pharmaceutical composition for use in the treatment of cancer, in particular of a cancer selected from Hodgkin lymphoma, and primary mediastinal non-Hodgkin lymphoma.

## BRIEF DESCRIPTION OF THE DRAWINGS

To complete the description and in order to provide for a better understanding of the disclosure, a set of drawings is provided. Said drawings form an integral part of the description and illustrate embodiments of the disclosure, which should not be interpreted as restricting the scope of the disclosure, but just as examples of how the disclosure can be carried out. The drawings comprise the following figures:

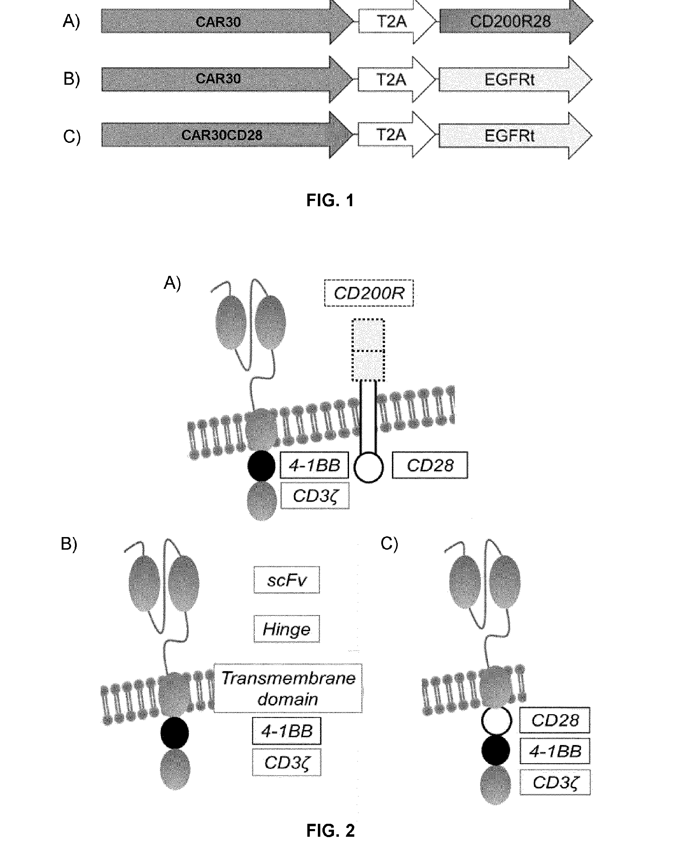

**Figure 1:** Schematic representation of protein expressing domains and regulatory domains in polynucleotide constructs expressing: A) a protein combination comprising a CAR (a) directed to CD30 and a modified CD200R (b) according to Example 1 (CAR30_CD200R28); B) a protein combination comprising a CAR (a) according to Comparative Example 2a (CAR30_ EGFRt); and C) a protein combination comprising a CAR (a) directed to CD30 additionally comprising a CD28 intracellular signaling domain according to Comparative Example 2b (CAR30CD28_ EGFRt).

**Figure 2:** Schematic representation of: A) a protein combination comprising a CAR (a) and a modified CD200R (b) according to Example 1 (CAR30 & CD200R28); B) a CAR directed to CD30 (a) according to Comparative Example 2a (CAR30); C) a CAR (a) directed to CD30 comprising a CD28 intracellular signaling domain according to Comparative Example 2b (CAR30CD28).

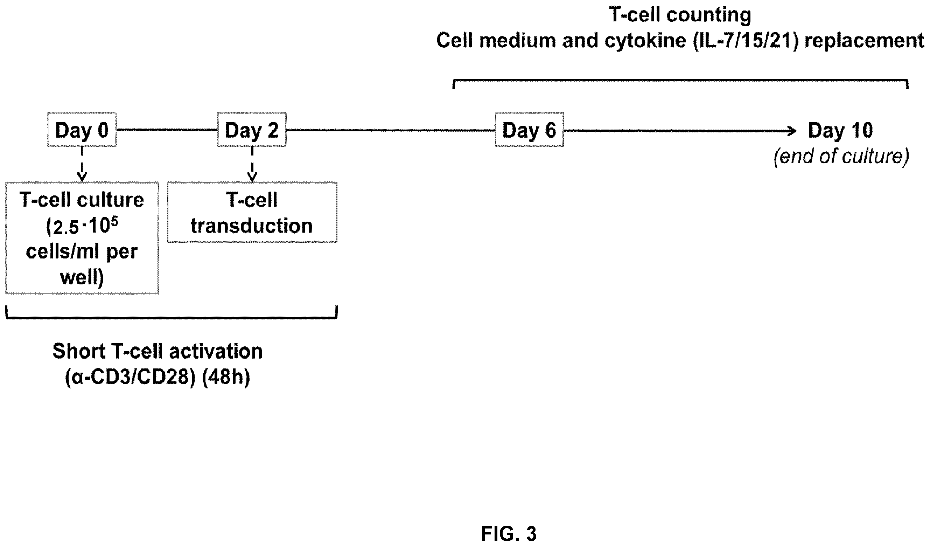

**Figure 3:** Schematic representation of the preparation of modified T-cells including T-cell activation, T-cell transduction, and T-cell expansion (according to Example 4).

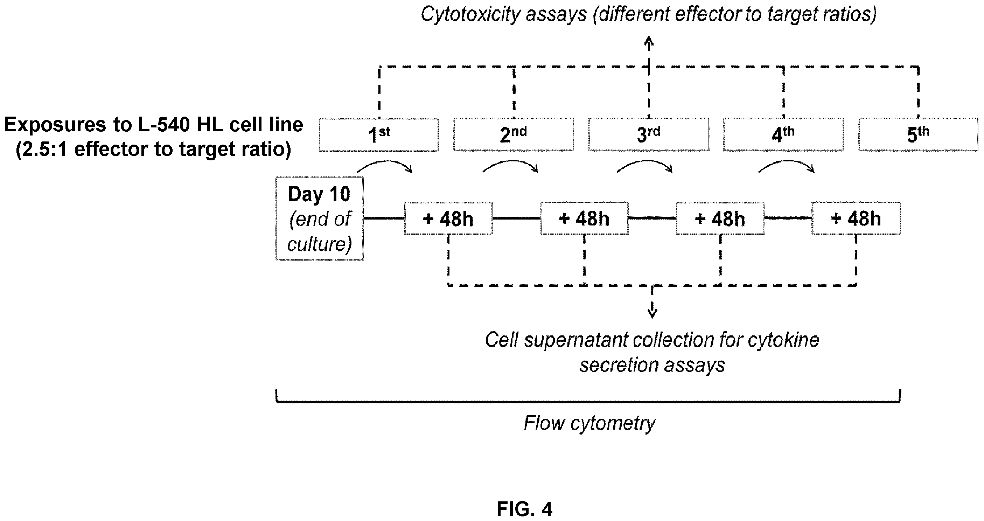

**Figure 4:** Schematic representation of cytotoxicity and cytokine secretion assays (of Example 5) showing 5 sequential exposures of the modified T-cells to tumor cells (of the L-540 HL cell line) at 48 h intervals.

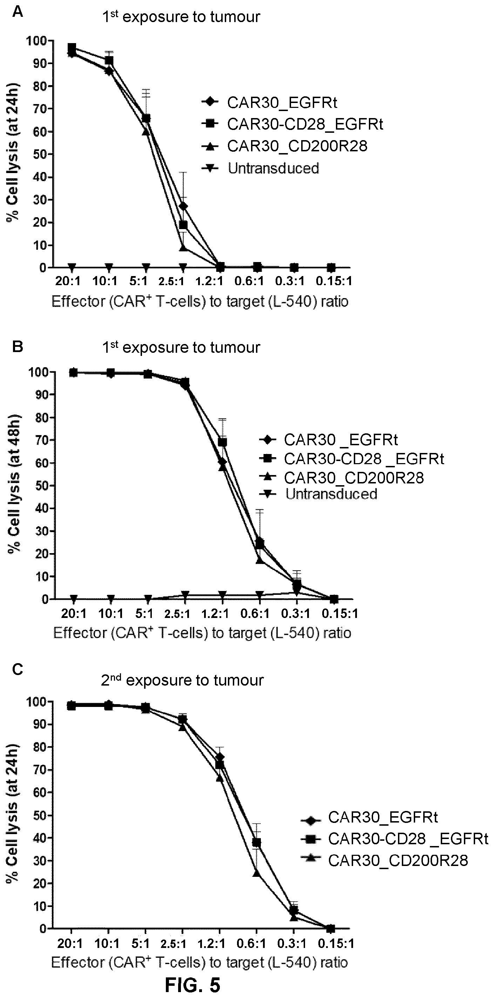

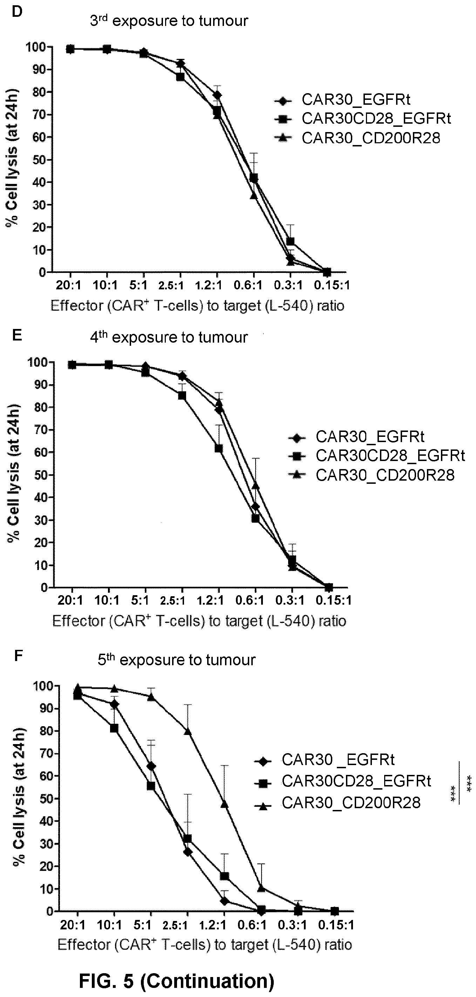

**Figure 5:** Graphical representations of the cytotoxicity assay results (of Example 5b) expressed in terms of % cell lysis at different effector (CAR+ T-cells) to target (L-540 HL tumor cells) ratios (E:T) 48h after a first or subsequent pre-exposures to tumor cells, cell lysis being measured 24 h or 48 h after exposure to the different E:T ratios: A) % of cell lysis after a 1st pre-exposure of modified immune cells to tumor cells (measured at 24 h); B) % of cell lysis after a 1st pre-exposure of modified immune cells to tumor cells (measured at 24 h); and % of cell lysis after a 2nd I, 3rd (D), 4th I and 5th (F) pre-exposure to tumor cells (all measured at 24 h). Measurements correspond to T-cells modified with a protein combination comprising a CAR (a) and a modified CD200R (b) according to Example 1 (CAR30_CD200R28,) (triangles, pointing upwards); T-cells modified with a CAR (a) according to Comparative Example 2a (CAR30_ EGFRt) (diamonds); T-cells modified with a CAR (a) comprising a CD28 intracellular signaling domain according to Comparative Example 2b (CAR30-CD28_EGFRt) (squares); and unmodified (untransduced) T-cells (triangles, pointing downwards). Data represent mean $\pm$ SEM of five independent healthy donors and *** means a p<0.0001 for the values being compared.

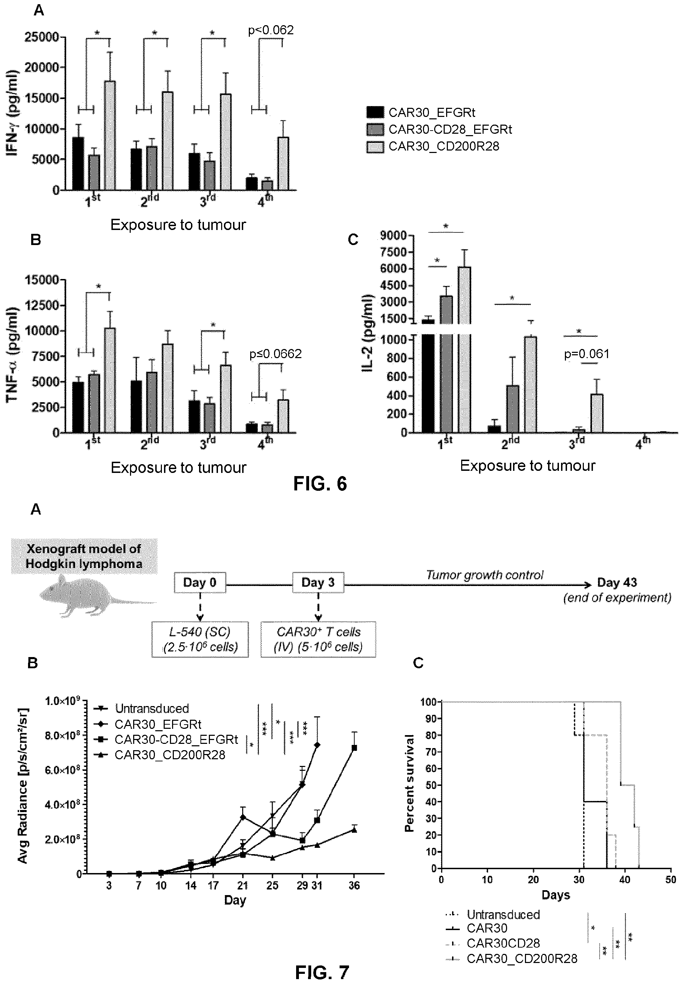

**Figure 6:** Graphical representations of the cytokine secretion (expressed as pg/ml of cytokine) assay results (of Example 5c) after a 1st, 2nd, 3rd and 4th pre-exposures to tumor cells measured for A) IFN-$\gamma$, B) TNF-$\alpha$ and C) IL-2 cytokines, for Example 1 (CAR30_CD200R28), Comparative Example 2a (CAR30_EGFRt), and Comparative Example 2b (CAR30-CD28_EGFRt). Data represent mean $\pm$ SEM of four independent healthy donors and * means a p<0,05,

**Figure 7:** Schematical representation of in vivo assays (of Example 6) (A) and graphical representation of the *in vivo* test results for CAR30_CD200R28 T-cells of Example 1 (triangles pointing upwards), CAR30_EGFRt T-cells of Comparative Example 2a (diamonds), CAR30-CD28_EGFRt, T-cells of Comparative Example 2b (squares), and untransduced T-cells (triangles pointing downwards), in terms of

- tumor growth control (B) by measurements of average Radiance vs. days A higher average radiance is indicative of a higher tumor growth, and
- survival % I

wherein * means p < 0.05; ** means p < 0.01; and *** means p < 0.001.

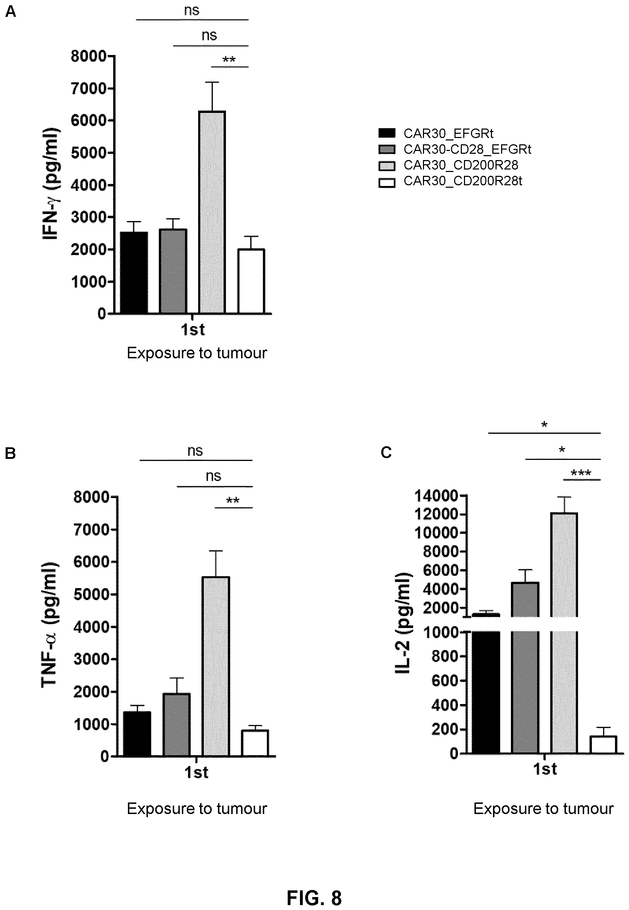

**Figure 8:** Graphical representations of the cytokine secretion (expressed as pg/ml of cytokine) assay results (of Example 5c) after a 1st exposure to tumor cells measured for A) IFN-γ, B) TNF-α and C) IL-2 cytokines, for Example 1 (CAR30_CD200R28), Comparative Example 2a (CAR30_EGFRt), Comparative Example 2b (CAR30-CD28_EGFRt) and Comparative Example 1 (CAR30_CD200R28t). Data represent mean ± SEM of four independent healthy donors and ns means non-signifcant difference, * means p < 0.05; ** means p < 0.01; and *** means p < 0.001

## DETAILED DESCRIPTION

The present invention particularly relates to a protein combination comprising a chimeric antigen receptor (CAR) (a) and a modified CD200 receptor (CD200R) (b).

The term "chimeric antigen receptor" or "CAR" or "CARs" (in plural) as used herein refers to engineered receptors, also referred in the art as "CAE", which graft an antigen specificity onto cells (for example immune cells such as T-cells or Natural Killer cells). CARs are also known as artificial T-cell receptors, chimeric T-cell receptors or chimeric immunoreceptors. In various embodiments, CARs are recombinant polypeptides. CARs can provide both antigen binding and immune cell activation functions onto an immune cell such as a T-cell, e.g., by inducing T-cell signaling.

A CAR (a) as described herein typically comprises:

iii. an ectodomain,
ii. an endodomain, and
iii. a transmembrane domain connecting the ectodomain and the endodomain,

wherein the ectodomain comprises an antigen binding domain that specifically binds an antigen selected from a CD30 antigen, a CD15 antigen, and a CD123 antigen.

A modified CD200R (b) in a protein combination as described herein is a recombinant receptorthat has the ability to bind to CD200 but upon binding to CD200, the response of the modified receptor is different from the inhibitory activity triggered upon binding to the wild-type CD200 receptor. For instance, upon binding to CD200, a modified CD200R of a protein combination as described herein, may have a reduced inhibitory activity, may have no inhibitory activity or may redirect the activity of the bound ligand to enhance T-cell function instead of inhibiting it.

A modified CD200R (b) as described herein typically comprises an extracellular domain, a transmembrane domain and an intracellular domain, wherein:

iv. the extracellular domain comprises a binding domain of CD200R, and optionally comprises an extracellular portion of CD28 connecting the binding domain of CD200R and the transmembrane domain,
v. the transmembrane domain is selected from a transmembrane domain of CD200R or a transmembrane domain of a protein other than CD200R, preferably the protein other than CD200R is CD28, and
vi. the intracellular domain is an intracellular stimulatory signaling domain, and preferably is an intracellular stimulatory signaling domain of CD28.

The terms "ectodomain", "endodomain", and "transmembrane domain" are used herein as commonly used in the art. The terms ectodomain and endodomain are often used interchangeably respectively with the terms "extracellular domain" and "intracellular domain." In the present description ectodomain and endodomain are used to refer to the domains of a CAR and extracellular domain and intracellular domain are used to refer to the domains of a modified CD200 receptor. Both the terms ectodomain and extracellular domain refer to the domain of the respective protein extending out to the extracellular space and endodomain and intracellular domain refer to the domain of the respective protein extending into the intracellular space. The "transmembrane" domains are the domains of the respective proteins that span the membrane of a cell.

### The CAR (a)

As indicated above, an ectodomain (i) of a CAR (a) described herein comprises an antigen binding domain that specifically binds an antigen selected from a CD30 antigen, a CD15 antigen, and a CD123 antigen. These antigens may be individually or collectively referred herein as target antigen or target antigens. CARs specifically binding the target antigens may also be referred to as CARs directed to the corresponding antigen (e.g., a CD30 directed CAR, *etc*...) or may simply be referred to by including the name of the protein of the antigen they are directed to (e.g., CD30-CAR or CAR30 for CD30; or CD15-CAR or CAR15 for CD15; or CD123-CAR or CAR123 for CD123).

Typically, the antigen binding domain is a molecule or a portion of a molecule, preferably a protein fraction of the ectodomain, which binds a target molecule (e.g., an antigen). Generally, the antigen binding domain may comprise a peptide or a protein, which is capable of binding to a target antigen selected from a CD30 antigen, a CD15 antigen, and a CD123 antigen. Such peptide or protein may be an antibody or a fragment of an antibody or a functional equivalent or a derivative thereof; T-cell receptors (TCRs) or portions thereof. For instance, antigen binding domain may comprise full length heavy chain, a fragment antigen-binding region (Fab region), single chain variable fragment (scFv), e.g., comprising heavy and light chain variable regions of a monoclonal antibodies connected by a peptide linker.

For instance, binding domains binding to a target antigen, such as a CD30 antigen, a CD15 antigen, and a CD123 antigen, maybe an antibody, e.g., a CD30 antibody, a CD15 antibody, a CD123 antibody, and a B-cell maturation (BCM) antibody, or a fragment of the corresponding antibody, such as a scFv of the corresponding antibody.

Identifying and preparing an antigen binding domain (e.g., an antibody) that specifically binds a targeted antigen is within the scope of a skilled person.

The term "specifically binds" as used herein, means that a polypeptide or protein fragment interacts more frequently, more rapidly, with greater duration, with greater affinity, or with some combination of the above to the epitope, protein, or target molecule than with alternative substances, including related and unrelated proteins.

For instance, an antigen binding domain (e.g., an antibody) that specifically binds an antigen may be known to a skilled person, e.g., from relevant literature, or may be identified, for example, by immunoassays, ELISAs, SPR (e.g., Biacore), or other techniques known to those of skill in the art. Typically a binding may be regarded as being specific, when the signal for the specific binding reaction is at least twice the background signal or noise, and can be more than 10 times the background signal. Reference is made to, e.g., Paul 2003 (Paul, ed., 2003, Fundamental Immunology Fifth Edition, Lippincott Williams & Wilkins; ISBN-10: 0781735149) for a discussion regarding antibody specificity. Also, Nagata *et al.* 2005 (Nagata et al., Cell membrane-specific epitopes on CD30: Potentially superior targets for immunotherapy., Proceedings of the National Academy of Sciences of the United States of America vol. 102, 22 (2005): 7946-51. doi:10.1073/pnas.0502975102) measure and disclose the affinities of different antibodies that specifically bind to CD30 antigen. A binding domain that specifically binds a target antigen can bind the target antigen at a higher affinity than its affinity for a different molecule. In some embodiments, a binding domain that specifically binds a target antigen can bind the target antigen with an affinity that is at least 20 times greater, at least 30 times greater, at least 40 times greater, at least 50 times greater, at least 60 times greater, at least 70 times greater, at least 80 times greater, at least 90 times greater, or at least 100 times greater, than its affinity for a different molecule. In some embodiments, a binding domain that specifically binds a particular target antigen binds a different molecule at such a low affinity that binding cannot be detected using an assay described herein or otherwise known in the art. In some embodiments, "specifically binds" means, for instance, that a binding moiety binds a target antigen with a dissociation constant (KD) of about 0.1 mM or less. In some embodiments, "specifically binds" means that a binding domain binds a target antigen with a KD of at about 10 $\mu$M or less or about 1 $\mu$M or less. In some embodiments, "specifically binds" means that a binding domain binds a target antigen with a KD of at about 0.1 $\mu$M or less, about 0.01 $\mu$M or less, or about 1 nM or less. The KD may be determined by methods known in the art such as enzyme-linked immunosorbent assay (ELISA), as described by, e.g., Friguet *et al.* (Friguet et al., Measurements of the true affinity constant in solution of antigen-antibody complexes by enzyme-linked immunosorbent assay. Journal of immunological methods vol. 77,2 (1985): 305-19. doi:10.1016/0022-1759(85)90044-4). Briefly, to determine the binding affinity of an antibody (e.g., a monoclonal antibody (Mab)) to a relevant antigen (e.g. CD30-Fc), the antibody may be incubated in solution with various concentrations of the relevant antigen at room temperature (e.g. 25 °C) for 20 h to reach equilibrium; then the amount of the antibody that remains unbound at equilibrium may be measured by an indirect ELISA using the relevant antigen as the coating antigen.

As such, "specific binding" does not necessarily require (although it can include) exclusive binding, i.e., binding to a single target. Thus, a binding moiety can, in some embodiments, specifically bind more than one target. For example, an antibody can, in certain instances, comprise two identical antigen-binding sites, each of which specifically binds the same epitope on two or more proteins. In certain alternative embodiments, an antibody can be bispecific and comprise at least two antigen-binding sites with differing specificities.

Amino acid sequences of antigen domains specifically binding the targeted antigens and nucleotide sequences encoding de same, may be known from the art.

In several embodiments, the antigen binding domain may comprise an antibody or a region of an antibody specifically targeted against an antigen selected from a CD30 antigen, a CD15 antigen, and a CD123 antigen, preferably against a CD30 antigen. More in particular, the antigen binding domain may comprise the variable regions of heavy and light chains of monoclonal antibodies specifically targeting an antigen of interest, yet more in particular may comprise single-chain variable fragments (scFvs) comprising heavy and light chain variable regions of a monoclonal antibodies specifically targeting an antigen of interest, connected by a peptide linker. In several particular embodiments, the antigen binding domain may comprise a scFv comprising the variable regions of heavy and light chains of a monoclonal antibody specifically targeted against an antigen selected from a CD30 antigen, a CD15 antigen, and a CD123 antigen, preferably against a CD30 antigen.

Suitable binding domains (and their respective amino acid sequences and nucleotide encoding sequences) may also be identified, e.g., using the sequence of a target antigen, as described in Nagata *et al.* 2005 and Nagata *et al.* 2002 (Nagata et al. Novel anti-CD30 recombinant immunotoxins containing disulfide-stabilized Fv fragments. Clinical cancer research: an official journal of the American Association for Cancer Research vol. 8,7 (2002): 2345-55).

For the identification of an appropriate antigen binding domain, it may be important to consider factors such as the affinity for its antigen (below a lower limit of affinity, antigen recognition does not efficiently induce T-cell activation; but, above an upper limit, nonspecific recognition of non-tumoral cells expressing lower densities of the antigen may occur. Also, above an upper limit, excessive activation of T cells may lead to exhaustion or activation induced cell death). Reference is made to, e.g., Stoiber *et al.* 2019 (Stoiber et al. Limitations in the Design of Chimeric Antigen Receptors for Cancer Therapy. Cells vol. 8,5 472. 17 May. 2019, doi:10.3390/cells8050472) and Chmielewski *et al.* 2004 (Chmielewski et al. T cell activation by antibody-like immunoreceptors: increase in affinity of the single-chain fragment domain above threshold does not increase T cell activation against antigen-positive target cells but decreases selectivity." Journal of Immunology, vol. 173,12 (2004): 7647-53. doi:10.4049/jimmunol.173.12.7647).

Besides, another important factor to consider when choosing an appropriate binding domain, may be the localization of the epitope. For example, if an epitope is located in the non-soluble region of an antigen, only the non-soluble antigen will be recognized by the antigen binding domain, the soluble antigen would not be recognized. In a particular embodiment, it may be advantageous for the epitope, e.g., of CD30, CD15, CD123 and BMCA to be located in the non-soluble region of any of said proteins. Therefore, the soluble protein could not inhibit CAR30 binding to membrane-anchored protein, after being released from tumor cells. This has been previously described in the case of CD30 (Nagata, *et al.* 2005).

A CAR (a) as described herein may comprise one or more antigen binding domains, e.g., two or more antigen binding domains. Two or more binding domains may be separated by, e.g., linker fragments, such as a Whitlow peptide linker as described by Whitlow *et al.* 1993 (Whitlow et al., An improved linker for single-chain Fv with reduced aggregation and enhanced proteolytic stability. Protein Eng. 1993 Nov;6(8):989-95. doi: 10.1093/protein/6.8.989).

If more than one binding domain are present, they may specifically bind to one or more different antigens of the same protein or of two or more different proteins, in particular may specifically bind to the same protein, for instance, they may bind to a CD30 antigen.

In several embodiments two antigen binding domains may correspond to a light chain and a heavy chain, two light chains, or two heavy chains, preferably a light chain and a heavy chain of an antigen binding antibody.

In several embodiments a CAR (a) as described herein may comprise two antigen binding domains. In several particular embodiments two antigen binding domains may bind to the same protein and more in particular may bind to CD30. For instance, a CAR (a) as described herein may comprise a heavy chain and a light chain of a CD30 antibody.

Binding domains binding a CD30 antigen are known. Further, CD30 biding domains may be identified as described above. Examples of CD30 binding domains may be include those disclosed in, e.g., International Application published as WO 2020/135559, Alvarez-Fernández *et al.* 2021 (Alvarez-Fernández et al., Memory stem T cells modified with a redesigned CD30-chimeric antigen receptor show an enhanced antitumor effect in Hodgkin lymphoma., Clin. Transl. Immunology 2021; 10(4):1268) and Alvarez-Fernández *et al. 2016* (Alvarez-Fernández et al., A short CD3/CD28 costimulation combined with IL-21 enhance the generation of human memory stem T cells for adoptive immunotherapy., Journal of translational medicine 2016; 14(1): 214), Wang *et al.* 2017 (Wang et al., Autologous T cells expressing CD30 chimeric antigen receptors for relapsed or refractory Hodgkin lymphoma: an open-label phase I trial., Clin. Cancer Res. 2017; 23: 1156-1166) and Ramos *et al.* 2020 (Ramos et al., Anti-CD30 CAR-T-cell therapy in relapsed and refractory Hodgkin lymphoma., J. Clin. Oncol. 2020; 38: 3794-3804).

The percentages of identity as defined herein may be of at least 95 %, in particular of at least 97%, more in particular of at least 99 %, even more in particular of at least 99.5 %, yet more in particular of at least 99.7%, even yet more in particular of at least 99.9 %, or even of at least 99.99%.

A CD30 protein may comprise or consist of amino acid sequence of SEQ ID **No. 1** or a variant thereof having a percentage of identity as defined above with the sequence of SEQ ID **No. 1,** preferably a percentage of identity of at least 99 %. Preferably, the antigen-binding domain of the invention binds to non-cleavable regions of SEQ ID **No. 1,** in particular to, e.g., amino acid residues 107-153 and 282-338 from SEQ ID **No. 1,** said CD30 binding domains were identified as membrane specific epitopes in, e.g., Nagata *et al.* 2005.

In particular embodiments, a binding domain specifically biding to a CD30 antigen may comprise or consist of the amino acid sequence of SEQ ID **No. 2** or a variant thereof having a percentage of identity as defined above with the sequence of SEQ ID **No. 2** and, e.g., said variant retaining the capacity to specifically bind CD30. Preferably a percentage of identity may be of at least 99%.

A CD15 protein may comprise or consist of amino acid sequence of SEQ ID **No. 3** or a variant thereof having a percentage of identity as defined above with the sequence of SEQ ID **No. 3,** preferably a percentage of identity of at least 99 %.

Binding domains binding a CD15 antigen are known. Further, CD15 biding domains may be identified as described above. Examples of CD15 binding domains include, e.g., those described in European Patent Application published as EP3455255. For instance, suitable CD15 binding domains may comprise the CDRs, light and heavy chain variable domains or other CD15 binding fragment of, e.g., CD15 antibody clone C3D-1, Carb-3 (DAKO A/S), MMA (CD15 binding fragment of Roche) or BY87 (Abeam).

A CD123 protein may comprise or consist of amino acid sequence of SEQ ID **No. 4** or a variant thereof having a percentage of identity as defined above with the sequence of SEQ ID **No. 4,** preferably a percentage of identity of at least 99 %.

Binding domains binding a CD123 antigen are known. Further, CD123 biding domains may be identified as described above. Examples of suitable CD123 binding domains may include, e.g., those described by Baroni *et al.* 2020 (Baroni et al., 41BB-based and CD28-based CD123-redirected T-cells ablate human normal hematopoiesis in vivo, J. Immunother. Cancer. 2020; 8(1): e000845), European Patent Application published as EP 3626247 or in International Application published as WO 2014/130635. For instance, suitable CD123 binding domains may comprise light and heavy chain variable domains or other CD123 binding fragments of anti-CD123 antibodies such as, e.g., CSL362, CSL360, 73G, or clone 6H6 (AbD Serotec).

The ability of a variant having a percentage of identity to an amino acid sequence of a binding domain that specifically binds an antigen, to retain the capacity to specifically bind the target antigen, may be assessed by methods known in the art, as also indicated above.

In particular, a suitable variant may retain a 100% of the capacity of the original sequence to specifically bind the target antigen, may retain at least an 80% of the specifically binding capacity of the original sequence, in particular at least a 90%, more in particular at least 95% and yet more in particular at least 99%. A suitable variant may also improve the capacity of the original sequence to specifically bind the target antigen. The capacity to specifically bind a targeted antigen of a variant with respect to the original sequence may be determined by measuring the binding affinity of the variant with respect to the target antigen of interest, as discussed above.

In several embodiments, in the CAR (a) the antigen binding domain of the ectodomain (i) preferably specifically binds a CD30 antigen. Targeting a CD30 antigen may be particularly interesting because, as described above, CD30 is a convenient target for Hodgkin and other CD30+ lymphomas.

More preferably the antigen binding domain of the ectodomain (i) may specifically bind an epitope within the non-cleavable part of CD30 receptor and more preferably the antigen binding domain. CARs specifically binding such non-cleavable part of CD30 have been previously described by, for instance, Alvarez-Fernández *et al.* 2021 and have been found to avoid the inhibition of CAR30-T cell cytotoxic capacity mediated by their interaction of these CAR30-T cells with the released soluble fraction of CD30.

Preferably, a binding domain biding to a CD30 antigen may comprise or consist of the amino acid sequence of SEQ ID **No. 2** or a variant thereof having a percentage of identity as defined above with the sequence of SEQ ID **No. 2** and, e.g., said variant retaining the capacity to bind an epitope within the non-cleavable part of CD30. Preferably a percentage of identity may be of at least 99 %. A binding domain specifically biding to a CD30 antigen may be preferably encoded by a sequence comprising or consisting of the nucleotide sequence of SEQ ID **No. 5.**

A protein sequence known to specifically bind an antigen of interest, may be incorporated into an ectodomain (i) of a CAR whereby the CAR is then directed to bind the specific antigen of interest, by methods known in the art. For instance, the sequence of a binding domain of a known CAR may be replaced by the sequence of the biding domain directed to the specific antigen of interest selected from the CD30 antigen, a CD15 antigen, and a CD123 antigen, and particularly, the specific antigen binding domains defined above. A recombinant CAR with the sequence of the antigen binding domain of interest may be then obtained by methods known in the art. For instance, a method may comprise: a 1st step for generating, e.g., hybridomas against a new target antigen, identifying the epitopes and determining the affinity; a 2nd step for generating, e.g., a scFv region in the CAR with some of the antibodies described; and a 3rd step for performing a functional analysis to choose the best scFv. For instance, CARs of the publications indicated above directed to CD30 (e.g., WO 2020/135559, Alvarez-Fernández *et al.,* 2021 and Alvarez-Fernández *et al.* 2016) may be used as a CD30 directed CAR of a protein combination as described herein or may be repurposed to provide a CD15, CD123 directed CAR of a protein combination as described herein.

A CAR described herein, having an ectodomain (i) comprising an antigen binding domain that specifically binds an antigen selected from a CD30 antigen, a CD15 antigen, and a CD123 antigenas defined above, may typically further comprise:

- an endodomain (ii) comprising a signaling module, optionally comprising a co-stimulatory module, and
- a transmembrane domain (iii) connecting the ectodomain (ii) and the endodomain (i).

A signaling module (e.g., of CD3ζ) of the endodomain (ii) may be useful to mediate downstream signaling during T-cell activation, which can be derived from the intracellular signaling portion of the T-cell receptor. A co-stimulatory module can enhance the activity of the CAR, e.g., by enhancing cytokine production, which can be derived from the intracellular signaling domains of the corresponding co-stimulatory proteins (e.g., 4-1BB and CD28).

The signaling module can be any signaling module known in the art as appropriate for mediating downstream signaling during T-cell activation. In some embodiments, the signaling module may be of, e.g., CD3ζ.

The signaling module may preferably be of CD3ζ, as this signaling module resembles the TCR-CD3 complex and the endogenous activation machinery of a T cell.

In some embodiments, the CD3ζ signaling module may comprise or consist of the amino acid sequence of SEQ ID **No. 6** or **7** a variant thereof having a percentage of identity of as defined above with the sequence of SEQ ID **No. 6** or **7;** may comprise a fragment thereof, in particular a fragment from residue 51 to 164 or from residue 52 to 164 of SEQ ID **No. 6** or a fragment from residue 2 to 113 of SEQ ID **No. 7,** or a fragment having a percentage of identity as defined above with such a fragment, and, e.g., said variant retaining the signaling capacity of CD3ζ. Preferably a percentage of identity may be of at least 99 %. A signaling module of CD3ζ may be encoded by, e.g., a sequence comprising or consisting of the nucleotide sequence of SEQ ID **No. 8** (corresponding to the human mRNA transcript variant 1 encoding the protein of SEQ ID No. 6) or of the nucleotide sequence of SEQ ID **No. 9** (encoding SEQ ID No. 7).

It is preferred for a co-stimulatory module to also be present in the endodomain (ii). If present the co-stimulatory module, may be of a protein selected from 4-1BB (also known as CD137 or TNFRSF9), CD28, OX40, ICOS, CD27, CD40, lymphocyte function-associated antigen-1 (LFA-1) , CD2, CD7, LIGHT, NKG2C, B7-H3, TNFRSF9, TNFRSF4, TNFRSF8, CD40LG, ITGB2, KLRC2, TNFRSF18, TNFRSF14, HAVCR1, LGALS9, CD83, and a ligand that specifically binds CD83. In several embodiments, the co-stimulatory module is of a protein selected from 4-1BB, CD28, OX40 and ICOS. Preferably the endodomain (ii) may comprise a co-stimulatory module of 4-1BB and/or a co-stimulatory module of CD28, and more preferably the endodomain (ii) may comprise a co-stimulatory module of 4-1BB.

Co-stimulatory modules of 4-1BB are known from the art. In some embodiments, the 4-1BB co-stimulatory module may comprise or consist of the amino acid sequence of SEQ ID **No. 10** or a variant thereof having a percentage of identity as defined above with the sequence of SEQ ID No. 10, or may comprise a fragment thereof, in particular a fragment from residue 214 to 255, or from residue 214 to 254 of SEQ ID No. 1**0** or a fragment having a percentage of identity as defined above with such a fragment, and, e.g., said variant retaining the co-stimulatory capacity of 4-1 BB. Preferably a percentage of identity may be of at least 99 %.

A co-stimulatory module of 4-1BB may be encoded by a sequence comprising or consisting of the nucleotide sequence of SEQ ID No. **11** (corresponding to a human mRNA transcript encoding the protein of SEQ ID No. 10) or of the nucleotide sequence of SEQ ID No. **12** (encoding residues 214 to 255 of SEQ ID No. 1**0)** or residues 1 to 123 thereof (encoding residues 214 to 254 of SEQ ID No. 10).

Co-stimulatory modules of CD28 are part of CD28 proteins and are known from the art. An example of a CD28 protein may comprise or consist of amino acid sequence of SEQ ID No. **13** or a variant thereof having a percentage of identity as defined above with the sequence of SEQ ID No. **13** and, e.g., said variant retaining the co-stimulatory capacity of CD28. Preferably a percentage of identity may be of at least 99 %. As a mode of example, a variant of SEQ ID No. **13** with a percentage of identity of, e.g., 99.1% may be a variant having glycine (G) instead of Leucine (L) in amino acid residues no. 186 and 187 of SEQ ID No. **13.** A CD28 protein may be encoded by a sequence comprising or consisting of the nucleotide sequence of SEQ ID No. **14** (corresponding to a human mRNA transcript variant 1 encoding the protein of SEQ ID No. **13).**

The ability of a variant having a percentage of identity to an amino acid sequence of a signaling or co-stimulatory module as described above, to retain the signaling or co-stimulatory capacity of the original sequence, may be assessed by methods known in the art. For instance, by measuring T-cell expansion, cytotoxic capacity, cytokine secretion, *in vivo* persistence or any other routinary experiments usually perform in the field.

In particular, a suitable variant may retain a 100% of the signaling or co-stimulatory capacity of the original sequence, may retain at least an 80% of the signaling or co-stimulatory capacity of the original sequence, in particular at least a 90%, more in particular at least 95%, and yet more in particular at least 99%. A suitable variant may also improve the signaling or co-stimulatory capacity of the original sequence. The capacity to the signaling or co-stimulatory capacity of a variant with respect to the original sequence may be determined by methods known in the art, e.g., measuring T-cell expansion, cytotoxic capacity, cytokine secretion, *in vivo* persistence, determining T-cell subpopulations and other phenotypical markers.

A co-stimulatory module of 4-1BB may advantageously confer higher persistence *in vivo,* which can be explained due to decreased exhaustion and differentiation of cells and up-regulation of pro-survival proteins. In contrast, CARs containing CD28 co-stimulation have been shown to respond to lower antigen levels, by inducing potent T-cell activation and rapid development of effector functions. However, this increase in signal strength may lead to higher exhaustion and activation-induced cell death (AICD) in CD28-containing CAR-T cells, this is avoided by using 4-1BB.

A co-stimulatory module of 4-1BB has been found to work particularly well in combination with signaling modules of CD3ζ. Accordingly, in several embodiments a CAR of a protein combination as described herein may preferably comprise an endodomain (ii) that comprises a signaling module of CD3ζ and a co-stimulatory module of 4-1BB. In particular, a CAR of a protein combination as described herein may comprise an amino acid sequence of SEQ ID No. 15 or a variant thereof having a percentage of identity as defined above with the sequence of SEQ ID No. 15, preferably a percentage of identity of at least 99 %.

As indicated above, a CAR (a) described herein comprises a transmembrane domain (iii) connecting the ectodomain (ii) and the endodomain (i). A transmembrane domain is a membrane-spanning protein domain. Because the interior of the lipid bilayer is hydrophobic, the amino acid residues in transmembrane domains may typically be hydrophobic. Besides connecting the ectodomain (ii) and the endodomain (i) the transmembrane domain anchors the CAR to the membrane of a cell. Upon binding to the target antigen the transmembrane domain may propagate a signal from the antigen binding domain of the ectodomain to the endodomain and in particular to the signaling module and, if present, co-stimulatory module of the endodomain. Furthermore, another important function of the transmembrane domain is to provide stability to the molecule and favoring its expression in T-cell surface.

A transmembrane domain (iii) of a CAR (a) as described herein may comprise a transmembrane domain of a protein selected from CD8, CD28, CD4, and CD3ζ, preferably the transmembrane domain may be of CD8 and more preferably of CD8α. Advantageously the use of transmembrane domains from CD8, and of CD8α in particular, rather than CD28 may be preferred due to reduced signal strength, leading to reduced activation-induced cell death while maintaining efficacy in treated mice.

Transmembrane domains of CD8 (in particular CD8α), CD4, CD28 and CD3ζ are known in the art. Reference is made to, for instance, Stoiber *et al.* 2019.

Advantageously, CD8α and CD28-derived transmembrane domains have been found to increase CAR expression levels and stability on T-cell surface. CD3ζ-derived transmembrane domains may be less preferred since they may induce potent T-cell activation without the need to associate to the endogenous TCR complex.

Preferably the CAR comprises a transmembrane domain of CD8α, and more preferably the transmembrane domain of CD8α comprises or consists of amino acid sequence of SEQ ID No. **16** or a variant thereof having a percentage of identity as defined above with the sequence of SEQ ID No. **16** and, e.g., said variant retaining the capacity of anchoring the CAR to the membrane of a cell, and in particular embodiments also retaining the capacity of propagating a signal to the endodomain. Preferably a percentage of identity may be of at least 99 %.

In certain embodiments, the transmembrane domain is a transmembrane domain of CD28. Transmembrane domains of CD28 are known in the art. Reference is made to for instance Alabanza *et al.* 2017 (Alabanza et al., Function of Novel Anti-CD19 Chimeric Antigen Receptors with Human Variable Regions Is Affected by Hinge and Transmembrane Domains., Mol. Ther. 2017;25(11):2452-2465, doi:10.1016/j.ymthe.2017.07.013). Preferably a CD28 transmembrane domain comprises or consists of amino acid sequence of SEQ ID No. **17** or a variant thereof having a percentage of identity as defined above with the sequence of SEQ ID No. **17** and, e.g., said variant retaining the capacity of anchoring the CAR to the membrane of a cell, and in particular embodiments also retaining the capacity of propagating a signal to the endodomain. Preferably a percentage of identity may be of at least 99 %.

In several embodiments the CAR may comprise a signal peptide. Preferably the signal peptide may be a CD8 signal peptide and more preferably a CD8α signal peptide. The signal peptide favors the translocation of the protein to the cellular membrane, e.g., helps to direct the protein combination of the invention to the cell membrane and may be later released. The signal peptide may be expressed together with the protein combination. In particular, if present it may be the first peptide starting from the N-terminus of a CAR as described herein, just upstream of the ectodomain. However, the signal peptide it is not part of the ectodomain or of the functional CAR and it may or may not remain bound to it. In several embodiments the signal peptide is eliminated, and the functional CAR of a protein combination as described herein does not include the signal peptide.

Preferably the signal peptide comprises or consists of amino acid sequence of SEQ ID No. **18** or a variant thereof having a percentage of identity as defined above with the sequence of SEQ ID No. **18** and, e.g., said variant retaining the capacity of directing the translocation of the protein to the cellular membrane. Preferably a percentage of identity may be of at least 99 %.

The ectodomain (i) of a CAR of a protein combination as described herein may additionally comprise domains or fragments other than the antigen binding domain. For instance, the ectodomain (i) may optionally comprise a hinge domain.

In several embodiments the ectodomain (i) comprises a hinge domain. A hinge domain, also referred to in the art as a spacer region and may be included as a region of the ectodomain (i) extending from the antigen-binding domain to the transmembrane domain. Such a hinge domain may be included to provide flexibility to the antigen-binding domain and may enhance recognition of the target antigen. The hinge regions may include but are not limited to Fc fragments of antibodies or fragments or derivatives thereof, hinge regions of antibodies or fragments or derivatives thereof, CH$_2$ regions of antibodies, CH$_3$ regions of antibodies, artificial spacer sequences or combinations thereof.

The presence of hinge domain and the nature of such a domain may depend on the antigen binding domain and the target antigen and/or the nature of the transmembrane domain. Given that a hinge domain may improve the binding of the antigen binding domain with the target antigen, it may be preferred for the ectodomain (i) to comprise a hinge domain.

The length of the hinge domain may vary. For instance, hinge domains having from 40 to 100 amino acids, in particular from 50 to 90, more in particular from 55 to 85, more in particular from 60 to 75 amino acid residues, have been found to work well in a CAR as described herein. However, the hinge domain may be longer or shorter depending, e.g., on whether the target is a proximal or distant epitope.

The presence of CD8$\alpha$ hinge and transmembrane domains were found to show a reduced signal strength and related inflammatory cytokine secretion and activation-induced cell death (AICD) than with CD28 hinge and transmembrane domains, while maintaining equal antitumor efficacy in mice. This could be probably associated to the greater propensity of CD28-based hinges to dimerize (Alabanza *et al.* 2017).

Preferably the hinge domain may be selected from a CD8 hinge domain, CD28 hinge domain, and an IgG hinge domain, such as a modified human IgG1 or IgG4 hinge domain, more preferably the ectodomain comprises a CD8 hinge domain or a CD28 hinge domain.

In certain embodiments, the hinge domain may be a hinge domain of CD8, yet more preferably the CD8 hinge domain may be a CD8$\alpha$ hinge domain. In several particular embodiments the CD8$\alpha$ hinge domain preferably comprises or consists of amino acid sequence of SEQ ID No. **19** or a variant thereof having a percentage of identity as defined above with the sequence of SEQ ID No. **19** and, e.g., said variant retaining the capacity of favoring binding of the antigen domain to the target antigen. Preferably a percentage of identity may be of at least 99 %.

In certain embodiments, the hinge domain may be a hinge domain of CD28, and preferably comprises or consists of amino acid sequence of SEQ ID No. **20** or a variant thereof having a percentage of identity as defined above with the sequence of SEQ ID No. **20** and, e.g., said variant retaining the capacity of favoring binding of the antigen domain to the target antigen. Preferably a percentage of identity may be of at least 99 %.

Generally, the hinge domain of the ectodomain may be of the same protein as the transmembrane domain or may be of a different protein than the transmembrane domain. It may be preferred for the hinge domain to be of the same protein as the transmembrane domain. In several embodiments the transmembrane domain may be transmembrane domain of CD8 and the hinge domain a hinge domain of CD8, preferably of CD8$\alpha$. In several alternative embodiments, the transmembrane domain may be transmembrane domain of CD28 and the hinge domain may be a hinge domain of CD28.

A CAR of a protein combination as described herein may preferably comprise:

- an ectodomain (i) that specifically binds a CD30 antigen and comprises a CD8 hinge domain and optionally a CD8 signal peptide;
- a transmembrane domain (iii) of CD8; and
- an endodomain (ii) that comprises a first stimulatory signaling module of CD3$\zeta$ and a second stimulatory signaling module of 4-1BB.

The hinge domain, the signal peptide (if present) and the transmembrane domain may preferably be of CD8$\alpha$.

Such combination of ectodomain (i) transmembrane domain (iii) and endodomain (ii) has been found to advantageously avoid tonic signaling and excessive T-cell activation after antigen stimulation that could potentially lead to CAR-T cell exhaustion, differentiation, AICD, and lack of persistence.

In several embodiments, a CAR of a protein combination as described herein may preferably comprise or consist of an amino acid sequence of SEQ ID No. **21** or a variant thereof having a percentage of identity as defined above with the sequence of SEQ ID No. **21** and, e.g., said variant retaining the capacity to bind a CD30 antigen and to induce T-cell signaling. Preferably a percentage of identity may be of at least 99 %.

Said CAR of a protein combination as described may be preferably encoded by a sequence comprising or consisting of the nucleotide sequence of SEQ ID No. **22**.

The ability of a variant having a percentage of identity to an amino acid sequence of a CAR (a) as described above, e.g., to retain the capacity to bind a CD30 antigen and to induce T-cell signaling may be assessed with respect to the original sequence, may be determined by methods known in the art. As a mode of example methods as described above to assess the ability of a variant to bind an antigen or to retain the signaling or co-stimulatory capacity, may be used. T-cell signaling may be evaluated by the analysis of the phosphorylation of signaling molecules such as Lck or AKT or through functional studies (cytokine secretion, cytotoxic capacity...), as described in, e.g., Katsarou et al. 2021 (Katsarou et al., Combining a CAR and a chimeric costimulatory receptor enhances T cell sensitivity to low antigen density and promotes persistence., Sci. Transl. Med. 2021; 13 (623): eabh1962, doi:10.1126/scitranslmed.abh1962) and Kamsma *et al.* 2018 (Kamsma et al., Single-Cell Acoustic Force Spectroscopy: Resolving Kinetics and Strength of T Cell Adhesion to Fibronectin., Cell Rep. 2018;24(11):3008-3016, doi:10.1016/j.celrep.2018.08.034).

## The modified CD200R (b)

A protein combination as described herein comprises a modified CD200 receptor (CD200R) wherein the modified CD200R comprises an extracellular domain, a transmembrane domain and an intracellular domain.

A as described above a modified CD200R (b) of a protein combination as described herein has an extracellular domain (iv) that comprises a binding domain of CD200R.

Amino acid sequences of extracellular domains of CD200R and nucleotide sequences encoding de same, may be known from the art. Reference is made to, for instance, WO 2016/141357.

As a mode of example, the amino acid sequence of a binding domain of CD200R may correspond to a binding domain of a wild-type sequence of CD200R or a variant thereof that specifically binds CD200.

A variant of the binding domain wild-type sequence of CD200R, may be a sequence that includes a few amino acids that are different and/or a sequence that has more or less amino acids, for instance a sequence that is shorter by a number of amino acids, e.g., shorter by 3, 6, 9 or 12 amino acids, preferably shorter by 9 or 12 amino acids, more preferably shorter by 9 amino acids. A sequence that is shorter may preferably be shorter in the C-terminus of the sequence, where the number of amino acids starting from the C-terminus is absent. By shortening the sequence of the binding domain of CD200R, the size of the extracellular domain of the modified CD200R may be varied to accommodate, e.g., an extracellular portion of the CD28 extracellular domain, as explained further below. The size of the extracellular domain may also thereby be adjusted to optimize the ability of the modified CD200R to enter the immunological synapse with cells expressing CD200. This may, in turn, ensure delivering a strong co-stimulatory signal, where an intracellular signaling domain is present.

In particular embodiments the binding domain of CD200R may be at least a fragment of the extracellular region of CD200R. For instance, such binding domain of CD200R may comprise or consist of the amino acid sequence of SEQ ID **No.23,** or a variant thereof having a percentage of identity as defined above with the sequence of SEQ ID No. **23** and, e.g., said variant retaining the capacity to specifically bind CD200. Preferably a percentage of identity may be of at least 99 %.

The capacity of the binding domain of CD200R to specifically bind CD200 may be measured by methods known in the art as detailed above for the binding domain of the CAR (a). Similar protocols can be used, and easily adapted or specific protocols are required for CD200 binding, for example, reference is made to protocols described in Katsarou *et al.* 2021.

A binding domain of CD200R may be encoded by a sequence comprising or consisting of the nucleotide sequence of SEQ ID No. **24** or SEQ ID No. **25.**

Optionally, in addition to a binding domain of CD200R the extracellular domain (iv) of the modified CD200R may comprise an extracellular portion of a protein other than CD200R, such as CD28, as detailed further below.

A modified CD200R (b) of a protein combination as described herein further comprises a transmembrane domain (v). Similar to the transmembrane domain of the CAR (a), the transmembrane domain of the modified CD200R is a membrane-spanning protein domain that anchors the modified CD200R to the membrane of a cell.

The transmembrane domain (v) may be selected from a transmembrane domain of CD200R or a transmembrane domain of a protein other than CD200R, preferably the protein other than CD200R may be CD28.

In particular embodiments, a transmembrane domain of CD200R may comprise or consist of the amino acid sequence of SEQ ID No. **26** or a variant thereof having a percentage of identity as defined above with the sequence of SEQ ID No. **26** and, e.g., said variant retaining the capacity anchoring the modified CD200R to the membrane of a cell. Preferably a percentage of identity may be of at least 99 %.

In other particular embodiments, a transmembrane domain of CD28 may comprise or consist of the amino acid sequence of SEQ ID No. **17** or a variant thereof having a percentage of identity as defined above with the sequence of SEQ ID No. **17** and, e.g., said variant retaining the capacity of anchoring the modified CD200R to the membrane of a cell, and in particular embodiments also retaining the capacity of propagating a signal to the endodomain. Preferably a percentage of identity may be of at least 99 %.

As indicated above, the extracellular domain (iv) may also optionally comprise an extracellular portion of a protein other than CD200R, e.g., in particular an extracellular portion of CD28. Such extracellular portion may connect the binding domain of CD200R and the transmembrane domain. The extracellular portion of the protein other than CD200R, e.g.,of CD28, may be useful to modulate the distance of the binding domain present in the extracellular domain with respect to the cell membrane. The modulation of CD200R28 size may further contribute to improving fitting in the immune synapse important, as already described by Oda *et al.* 2017. Preservation of the CD28 cysteine in the extracellular domain may also contribute to inducing the dimerization with endogenous CD28, what can enhance T-cell activation. The presence of an extracellular portion of CD28 may be particularly preferred where the transmembrane domain (v) is of CD28. The presence of such extracellular portion of CD28 may further facilitate signal transduction from the binding domain of CD200R to the transmembrane domain where the transmembrane domain is of CD28.

The extracellular portion of CD28 may preferably be of a length from 9 to 12 amino acids (e.g., 9 amino acids or 12 amino acids), and in some aspects including a membrane-proximal-most cysteine residue of a CD28 extracellular region, preferably may be of a length of 12 amino acids, so that the first cysteine in the extracellular region is included.

In several particular embodiments the extracellular portion of CD28 may correspond to amino acids 141-152 of the wild-type sequence of CD28 SEQ ID No. **13,** which corresponds to the 12 amino acids of SEQ ID No. **27.**

The modified CD200R may optionally comprise an intracellular domain (vi).

An intracellular domain may be an intracellular stimulatory signaling domain and may preferably be an intracellular stimulatory signaling domain of CD28. Amino acid sequences of intracellular stimulatory signaling domain of CD28 and nucleotide sequences encoding the same, may be known from the art. Reference is made to, for instance, WO 2016/141357.

In particular embodiments, the intracellular stimulatory signaling domain of CD28 may comprise or consist of the amino acid sequence of SEQ ID No. **28** or a variant thereof having a percentage of identity as defined above with the sequence of SEQ ID No. **28** and, e.g., said variant retaining the capacity of intracellular stimulation of CD28. Preferably a percentage of identity may be of at least 95 %, more preferably at least of 99 %. As a mode of example, a variant of SEQ ID No. **28** with a percentage of identity of, e.g., 95.1 % may be a variant having glycine (G) instead of Leucine (L) in amino acid residues no. 7 and 8 of SEQ ID No. **28.**

The capacity of the intracellular domain of the modified CD200R (b) of intracellular stimulation may be measured by methods known in the art as detailed above for the signaling or co-stimulatory module of the endodomain of the CAR (a). Reference is made to, e.g., methods described in Katsarou *et al.* 2021.

If an intracellular stimulatory signaling domain is present in the modified CD200R (b), upon binding the extracellular domain to the target CD200 the transmembrane domain of modified CD200R (b) may propagate a signal from the antigen binding domain of the extracellular domain to the intracellular stimulatory signaling domain. Thereby the inhibitory signal of a CD200 protein upon binding to a CD200R may be turned into a stimulatory signal upon binding to a modified CD200R that has an intracellular co-stimulatory signaling domain.

In several particular embodiments, in the modified CD200R (b) of a protein combination as described herein:

- the protein other than CD200R of the transmembrane domain (v) is CD28,
- optionally, the extracellular domain (iv) comprises an extracellular portion of CD28 connecting the binding domain of CD200R and the transmembrane domain, and
- the intracellular domain is an intracellular stimulatory signaling domain of CD28.

A modified CD200R comprising a CD28 transmembrane domain, preferably in combination with an extracellular portion of CD28 and an intracellular stimulatory signaling domain of CD28, has been found to work particularly well with a CAR (a) of a protein combination as described herein. Without being bound to any theory, it is believed that the fact that the modified CD200R targets CD200 and that the CAR (a) targets antigens which are typically concomitantly expressed in cancerous cells with CD200, the stimulatory signals triggered by the binding of CD200 to the fusion CD200R protein may act together, with the stimulatory signals triggered by the binding of the antigen to the corresponding CAR binding domain, resulting in enhanced anticancer activity, e.g., of immune cells modified to express a protein combination as described herein. In several embodiments the combined effect of the CAR (a) and the modified CD200R (b) of the protein combination as described herein may be synergistic.

In several particular embodiments, in the modified CD200R (b) of a protein combination as described herein is a fusion CD200R protein.

A fusion protein is understood as a protein having domains of CD200R and one or more domains of at least one protein different from CD200R, preferably of CD28.

In particular embodiments, in a fusion CD200R protein as described herein:

- the extracellular domain (iv) may comprise a binding domain of CD200R, optionally comprising extracellular portion of CD28
- the transmembrane domain (v) may be a transmembrane domain of CD28 and
- the intracellular domain (vi) may be an intracellular stimulatory signaling domain of CD28.

In several preferred embodiments, the CD200R fusion protein comprises:

- an extracellular domain (iv) comprising a binding domain of CD200R and an extracellular portion of CD28,
- a transmembrane domain of CD28 and
- an intracellular stimulatory signaling domain of CD28.

What is described above for the preferred embodiments of the extracellular domain and in particular the extracellular portion, the transmembrane domain and the intracellular stimulatory signaling domain of CD28 also apply here.

Preferably the fusion CD200R protein may comprise or consist of the amino acid sequence of SEQ ID No. **29** or a variant thereof having a percentage of identity as defined above with the sequence of SEQ ID No. **29** and, e.g., said variant retaining the capacity of binding to CD200 and preferably also retaining the capacity of intracellular stimulatory signaling. Preferably a percentage of identity may be of at least 99 %. As a mode of example, a variant of SEQ ID No. **29** with a percentage of identity of, e.g., 99.4 % may be a variant having glycine (G) instead of Leucine (L) in amino acid residues no.

280 and 281 of SEQ ID No. **29.**

The capacity of the modified CD200R (b) of binding to CD200 and of intracellular stimulation may be measured by methods known in the art as detailed above.

## CD30-CAR and modified CD200R encoding nucleotides and vectors comprising the same

The present invention also relates to a polynucleic acid or a polynucleic acid combination comprising:

a. a first polynucleotide encoding the CAR (a), and
b. a second polynucleotide encoding the modified CD200R (b)

of the protein combination as described therein,
wherein the polynucleic acid comprises the first and second polynucleotide as part of a single polynucleic acid molecule and the polynucleic acid combination comprises the first and second polynucleotide as two independent polynucleic acid molecules.

A first polynucleotide encoding the CAR (a) may comprise or consist of the nucleotide sequence of SEQ ID No. **22** or a variant thereof having a percentage of identity as defined above with the sequence of SEQ ID No. **22** and, e.g., said variant encoding a CAR (a) retaining the capacity to specifically bind a CD30 antigen and to induce T-cell signaling. Preferably a percentage of identity may be of at least 99 %.

A second polynucleotide encoding the modified CD200R (b) may comprise or consist of the nucleotide sequence of SEQ ID No. **30,** or a variant thereof having a percentage of identity as defined above with the sequence of SEQ ID No. **30** and, e.g., said variant encoding a modified CD200R (b) retaining the capacity to bind CD200. Preferably a percentage of identity may be of at least 99 %.

A polynucleic acid comprising the first and second polynucleotide as part of a single polynucleic acid molecule may advantageously facilitate the simultaneous expression of the CAR (a) and the modified CD200R (b) so that they can be expressed at similar levels Liu, *et al.* 2017 (Liu et al., Systematic comparison of 2A peptides for cloning multi-genes in a polycistronic vector, Sci Rep 7, 2193 (2017), doi:10.1038/s41598-017-02460-2). The level of expression may be determined by methods known in the art. For instance, by RNA expression, Western blot or flow cytometry of the protein codified by the polinucleotide Preferably by flow cytometry using standard protocols known in the field.

A polynucleic acid combination comprising the first and second polynucleotide as two independent polynucleic acid molecules may advantageously facilitate the independent expression of the CAR (a) and the modified CD200R (b) so that they the level of expression of each protein may be modulated. However, co-transduction with two independent polynucleic acid molecules may have the inconvenience that T-cell products may not be homogeneous and single CAR or CD200R28 may be preferentially expressed in some T cells.

In several particular embodiments, a polynucleic acid comprising the first and second polynucleotide as part of a single polynucleic acid molecule may further comprise a 2A sequence, in particular a T2A or a T2A-like sequence or a P2A or a P2A-like sequence, preferably T2A or a T2Alike sequence, connecting the first and the second polynucleotide.

Such 2A sequences are regions encoding 2A peptides, e.g., a T2A or a T2A-like peptide or a P2A or a P2A-like peptide. Preferably the 2A peptide may be a T2A peptide. 2A peptides are self-cleaving peptides and is a class of 18-22 aa-long peptides, which can induce ribosomal skipping during translation of a protein in a cell and are found in a wide range of viral families. 2A peptides have been widely used to improve multigene co-expression efficiency in bi- and multi-cistronic constructs, in comparison to that obtained with other different strategies (Liu *et al.* 2017).

The incorporation of a 2A peptide in the single polynucleic acid molecule as described herein, e.g., selected from a T2A or a T2A-like or P2A or a P2A-like peptide, preferably a T2A peptide, has been found to improve the co-expression of the CD30-CAR and the modified CD200R.

A T2A-like sequence or a P2A-like sequence refers to a polynucleotide sequence that encodes a peptide that behaves like a T2A peptide and a P2A peptide respectively. Only one promoter is necessary to allow for the transcription of the whole polynucleotide sequence. However, although the mechanism is not yet fully elucidated, T2A sequence (as the rest of 2A sequences) is supposed to induce ribosomal "skipping" of glycyl-prolyl peptide bond formation during protein transduction, therefore giving rise to two different and independent proteins.

In several embodiments the single polynucleic acid molecule may comprise from 5' to 3': the first polynucleotide encoding the CAR (also referred to as the CAR encoding construct); followed by the region encoding the 2A peptide (preferably a T2A or a P2A peptide, more preferably a T2A peptide); and subsequently followed by the second polynucleotide encoding the modified CD200R (also referred to as the CD200R encoding construct).

Preferably the single polynucleic acid molecule may comprise or consist of the amino acid sequence of SEQ ID No. **31** or a variant thereof having a percentage of identity as defined above with the sequence of SEQ ID No. **31** and, e.g., said variant encoding a CAR (a) retaining the capacity to specifically bind a CD30 antigen and to induce T-cell signaling and a modified CD200R (b) retaining the capacity to bind CD200. Preferably a percentage of identity may be of at least 99 %.

The amino acid sequence codified by said polynucleotide corresponds to polypeptide of SEQ ID **No.32.**

The present invention further relates to a vector or a vector combination respectively comprising the polynucleic acid or the polynucleic acid combination as described therein, wherein the vector comprises the first and second polynucleotide as a single polynucleic acid molecule and the vector combination comprises a first vector comprising the first polynucleotide and a second vector comprising the second polynucleotide each independently.

A "vector" is a nucleic acid molecule that is capable of transporting another nucleic acid. Vectors may be, for example, plasmids, cosmids, viruses, or phages.

A vector or a vector combination as described herein may particularly refer to transfer vectors, to generate viral or non-viral vectors, or to viral or non-viral vectors as such, e.g., modified using such transfer vectors.

A vector or vector combination as described herein is useful for the introduction of the polynucleic acid or the polynucleic acid combination as described herein in target cells. Target cells may preferably be immune cells as described further below.

In several embodiments the vector of the vector or a vector combination as described herein may be a viral vector or a non-viral vector.

A non-viral vector may preferably be a transposon, and more preferably is a sleeping beauty transposon system.

The vector may preferably be a viral vector. There are a large number of suitable known viral vectors, including those identified for human gene therapy applications (see Pfeifer and Verma, Ann. Rev. Genomics Hum. Genet. 2: 177, 2001). Examples of suitable viral vectors include, e.g., vectors based on RNA viruses, such as retrovirus-derived vectors, e.g., Moloney murine leukemia virus (MLV)-derived vectors, and include more complex retrovirus-derived vectors, e.g., lentivirus-derived vectors. HIV-I-derived vectors belong to this category. Other examples include lentivirus vectors derived from HIV-2, FIV, equine infectious anemia virus, SIV, and Maedi-Visna virus (ovine lentivirus).

Preferably a viral vector as described herein may be a retroviral vector and more preferably is a lentiviral vector.

A vector comprising the first and second polynucleotide as a single polynucleic acid molecule may advantageously facilitate simultaneous transduction of the first and second polynucleic acid in the target cells.

A vector combination comprising a first vector comprising the first polynucleotide and a second vector comprising the second polynucleotide each independently may be used although may not be preferred, since it may lead to heterogeneous CAR-T cell products. However, if high genetic cargos are required, it could be used if, e.g., the use of transposons or crispr-Cas9 are not feasible options.

It may be preferred to use a vector comprising the first and second polynucleotide as a single polynucleic acid molecule.

In addition to the first and second polynucleotides, a vector (e.g. a transfer vector or resulting viral or non-viral vectors) may further comprise a reporter gene, e.g., encoding a protein which may be easily monitored, in order to assess the activity of the vector and the success of protein expression. Such reporter gene may be a gene encoding, e.g., a truncated Epidermal Growth Factor Receptor (EGFRt) or a green fluorescent protein (GFP). Nonetheless, in a vector or a vector combination, the first nucleotide encoding the CAR (a) and/or the second nucleotide encoding the modified CD200R (b) may be used as reporter gene.

Suitable transfer vectors may be prepared (e.g., synthesized and subcloned) by methods known in the art. For instance, the first and second polynucleotide encoding CAR (a) and modified CD200R (b) respectively, may be the synthesized and subcloned into a viral or a non-viral construct, e.g., a viral vector backbone. Viral and non-viral vector constructs, e.g., viral backbones, are commercially available.

As a mode of example, a viral construct may be a viral backbone vector that incorporates the first and second polynucleotide sequences to generate the transfer vector for later use in viral production. For instance, the viral backbone may be a retroviral vector backbone such as a lentiviral vector backbone (such as a replication-incompetent third generation (pHIV7), by GenScript Biotech (Piscataway, New Jersey, USA), or such as pMSGV, pSLCAR, pHR, or any other vector backbone commonly use in the field.

A non-viral vector construct may be, e.g., transposons (Sleeping Beauty or PiggyBac), RNA electroporation.

A transfer vector or a transfer vector combination may be used to generate viral or non-viral vectors. Methods of using viral vectors, in particular retroviral and more in particular lentiviral viral vectors, and packaging cells for transducing target cells with viral particles containing polynucleic acids of interest are known in the art and have been previously described in, for example, U.S. Patent 8,119,772 or Alvarez-Fernández *et al.* 2021).

For instance, for the preparation of viral vectors, a transfer vector or a transfer vector combination may be co-transduced into packaging cells (e.g., a Human Embryonic Kidney (HEK) cell line such as Lenti-X™ 293T-cells, which are commercially available from, for instance, Clontech Laboratories, Inc., Mountain View, California, USA) together with packaging vectors (e.g., pCHGP-2 codifying for gag and pol genes; and pCMV-rev2 codifying for rev gene) and envelope vectors (e.g., pCMV-VSV-G codifying for the Vesicular Stomatitis Virus envelope Glycoprotein, which are commercially available, e.g., from Addgene, Watertown, Massachusetts, USA).

Transfer, packaging, and envelope vectors can be previously amplified (e.g., in competent *Escherichia coli* such as Stbl3™ from Thermo Fisher, Germany) and purified using commercially available kits (such as HiSpeed® Plasmid Maxi Kit from Qiagen, Germany), according to manufacturer's instructions.

Co-transfection may be performed using commercially available transfection kits (e.g., CalPhos™ Mammalian Transfection Kit; Clontech Laboratories, Inc., Mountain View, California, USA).

After co-transfection packaging cells may be incubated for a sufficient amount of time (e.g., from 24 to 72 h, more in particular from 36 to 48h) to produce the viral vectors (or viral particles). The viral vectors may then be isolated and purified by means known in the art. As a mode of example the super natant of packaging cell culture may be collected and centrifuged to remove cellular debris (for, e.g., 5 to 30 minutes, in particular from 10 to 20 minutes at, e.g., 2500 to 5000 rpm, in particular at 3000 to 4000 rpm) and the supernatant may be subsequently ultracentrifuged to concentrate viral particles, e.g., lentiviral particles (for, e.g., 1 to 4 hours, from 1.5 to 3 hours at, e.g., 15000 to 35000 rpm, in particular from 20000 to 30000 rpm). The pellet of viral particles obtained may be snap-frozen and stored (e.g., -80 °C) to provide frozen viral particles for their subsequent use.

In a method as described herein the number of viral particles capable transduction of target cells was found to be from $1·10^6$ to $1·10^9$ transducing units per milliliter (TU/ml), in particular from $1·10^1$ to $5·10^8$. The transducing units per milliliter, also referred to as the viral titer, may be measured by methods known in the art. With viral vectors as described herein the viral titer may be measured by transducing tumor cells (e.g., Jurkat cells) with different volumes of a suspension of the viral particles (viral vectors) obtained and, after incubation of the transduced cells (e.g. for 24 to 72h, such as for 48 h), quantifying the number of cells expressing CD30-CAR on the cell membrane of the cells (also referred to as CAR30$^+$ cells), by staining and quantifying the fraction of CAR30+ cells by flow cytometry.

Viral titer may then be expressed in Transducing Units per milliliter (TU/ml) using the following formula:

$$TU/ml = \frac{(Total\ number\ of\ tumor\ cells) \cdot (\%\ CAR30^+\ cells)}{(Volume\ of\ virus\ that\ transduces\ aproximately\ 50\%\ of\ tumor\ cells)}$$

Vectors or vector combinations comprising a polynucleic acid or a polynucleic acid combination of interest as described herein, may be subsequently used for transducing target cells, e.g., immune cells, with such vectors. Thereby modified target cells (e.g., immune cells) may be obtained comprising said vectors, polynucleic acids and/or protein combinations as described herein.

**Modified immune cells**

The instant invention further relates to an immune cell comprising the protein combination as described herein, or the polynucleic acid or polynucleic acid combination as described herein and/or the vector or vector combination as described herein.

Such immune cells have been found useful in the treatment of cancer as indicated above as described in more detail below. Immune cells as described herein may have one or more of a protein combination, a polynucleic acid or polynucleic acid combination and/or a vector or vector combination as described herein.

For instance, since the vector or vector combination is used to introduce the polynucleic acid or polynucleic acid combination into target cells, and immune cells in particular, an immune cell comprising the vector or vector combination described herein typically will also comprise the polynucleic acid or polynucleic acid combination as described herein. Further, since the target cell may then use the polynucleic acid or polynucleic acid combination to express the corresponding protein combination, immune cells comprising a protein combination as described herein will also comprise the polynucleic acid or polynucleic acid combination and may also further comprise the vector or vector combination. At some stages, immune cells may not yet have expressed the protein combination but may comprise the vector or vector combination and the polynucleic acid or polynucleic acid combination of interest.

An immune cell as described herein may preferably be a T-cell or a natural killer (NK) cell.

A T-cell is an immune system cell that matures in the thymus. T-cells can be naive (not exposed to antigen), memory T-cells ($T_M$) (antigen- experienced and long-lived), and effector cells (antigen-experienced, cytotoxic). TM can be further divided into subsets of central memory T-cells ($T_{CM}$) and effector memory T-cells ($T_{EM}$). A subset of TM cells also includes T memory stem cells ($T_{SCM}$), which are a stem cell-like memory T-cell population capable of self-renewal and also capable of generating both the TCM and TEM subpopulations.

A natural killer (NK) cell, also known as large granular lymphocyte (LGL), is a type of immune cell that has granules (small particles) with enzymes that can kill tumor cells or cells infected with a virus.

Preferably the immune cell may be a T-cell or more preferably the T-cell may be a memory T-cell, and in particular selected from, e.g., a memory stem T-cell ($T_{SCM}$), a central memory T-cell ($T_{CM}$) and effector memory T-cell ($T_{EM}$), and yet more preferably is a combination of $T_{SCM}$ and $T_{CM}$

## Methods of manufacture

The instant invention further relates to a method for manufacturing an immune cell as described herein. The method for manufacturing a modified immune cell is not practiced on a human body or an animal body. Such a method is carried out ex *vivo* and/or *in vitro*.

The terms *"in vitro"* and *"ex vivo"* refer to the method of the invention being performed outside of the subject's body. That is, it is performed on a biological sample from a subject.

The term "subject" as used herein refers to a human or non-human animal, in particular a mammal such as a primate, horse, dog, mouse, or rat. The term "patient" as used herein refers to a "subject" which has been subjected to a medical treatment for the maintenance or amelioration of the health or to prophylactic treatments for preventing the degradation of health. Accordingly, the term "subject" encompasses a "patient" as defined above.

An *in vivo* / *ex vivo* method for manufacturing an immune cell as described herein may typically comprise:

a) isolating immune cells comprising naive T-cells and/or bulky CD4/CD8 T-cells from a biological sample which had previously been isolated from a subject to provide isolated immune cells,
b) culturing the isolated immune cells to provide immune cells comprising memory T-cells,
c) transducing, and optionally, culturing, the immune cells comprising memory T-cells obtained in step b) with a vector or a vector combination as described herein to provide an immune cell comprising the vector or vector combination of claim, the polynucleic acid or polynucleic acid combination of any one of claims and/or the protein combination as described herein.

As a person skilled in the art understands methods as described herein will typically lead to a plurality of modified immune cells. Accordingly, when referring to "an" immune cell or "a" modified immune cell a plurality of (modified) immune cells is also encompassed.

In step a) the immune cells comprising naive T-cells and/or bulky CD4/CD8 T-cells are isolated from a biological sample which had previously been isolated from a subject. The method of manufacturing the immune cell is therefore performed ex *vivo* / *in vitro*.

For purposes herein, the term "biological sample" may include, without limitation, whole blood or its derivatives such as serum, plasma, platelets, erythrocytes, and leukocytes. Also, the biological sample may include single cells or a plurality or fragments thereof. Preferably, the biological sample is whole blood or peripheral blood mononuclear cells (PBNCs). Naive T-cells and/or bulky CD4/CD8 T-cells are preferably isolated from whole blood or from peripheral blood mononuclear cells (PBMCs) which had previously been isolated from a subject.

The subject from which the biological sample (and the whole blood or the PBMCs in particular) may have been previously isolated may be a healthy subject or may be a subject suffering from, e.g., cancer, and more in particular a cancer selected from Hodgkin lymphoma, and primary mediastinal non-Hodgkin lymphoma.

The immune cells may be isolated, e.g., by enriching the naive T-cells and/or bulky CD4/CD8 T-cells to provide isolated immune cells by methods known in the art. For instance, commercially available isolation kits (e.g., EasySep™ Human T-cell Isolation Kit from StemCell Technologies, Vancouver, British Columbia, Canada), may be used according to the manufacturer's instructions.

The isolated immune cells obtained may typically comprise at least an 80 % cell count of naive T-cells and/or bulky CD4/CD8 T-cells from the total cell count, preferably at least an 85 %, more preferably a 90 % cell count of naive T-cells and/or bulky CD4/CD8 T-cells, from the total cell count. The count of naive T-cells and/or bulky CD4/CD8 T-cells may be performed by, e.g., flow cytometry. The staining of isolated T cells with antibodies against: CD3, CD4, CD8, CCR7, CD45RO, CD45RA, CD27 and CD95, to determine T-cell subpopulations after T-cell isolation from PBMCs. Analysis by flow cytometry may be performed as known in the art, e.g., as previously described by Lugli *et al.* 2013 (Lugli et al., Identification, isolation and in vitro expansion of human and nonhuman primate T stem cell memory cells., Nature protocols vol. 8,1 (2013): 33-42. doi:10.1038/nprot.2012.143)

In a method as described herein, an immune cell isolated from a biological sample previously isolated from a subject is converted into a non-natural or recombinant cell (e.g., a non-natural or recombinant T-cell) by introducing a polynucleic acid or polynucleic acid combination that encodes a protein combination as described herein and whereby the cell expresses the protein combination, which itself is non-natural. Such cells are also referred to herein as modified immune cells.

In step b) culturing isolated immune cells to provide immune cells comprising memory T-cells, may particularly comprise culturing the isolated immune cells obtained in step a) with anti CD3/CD28 co-stimulation in the presence of IL-7, IL-15 and IL-21, to provide immune cells comprising memory T-cells. Anti CD3/CD28 co-stimulation in the presence of IL-7, IL-15 and IL-21 may be performed by methods known in the art. For instance, the isolated immune cells may be cultured in the presence of CD3/CD28 magnetic beads, such as $\alpha$-CD3/CD28 magnetic beads (Dynabeads™; Life Technologies, Waltham, Massachusetts, USA) in a bead to T-cell ratio from 1:1 to 1:4, in particular from 1:1.5 to 1:3, and more in particular of 1:2 as previously described (Alvarez-Fernández *et al.,* 2016) Culturing in the presence of CD3/CD28 magnetic beads may be performed for 12 to 72 hours, in particular for 24 to 60 hours and yet more in particular for about 48 hours.

The immune cells obtained comprising memory T-cells, preferably comprise memory T-cells including $T_{SCM}$, $T_{CM}$ and $T_{EM}$, by the selection of bulk T cells and yet more preferably by the selection of TN cells. Early-memory T cells comprise $T_{scm} + T_{scm-like} + T_{cm}$ cells. These cells are enriched by short-activation of T cells with $\alpha$-CD3/CD28 and by the culture of these cells in the presence of IL-7, IL-15 and IL-21. Importantly, this procedure can be performed with bulk T cells but, preferably, TN selection is recommended.

The determination of early-memory subpopulations is performed as already described (flow cytometry according to, e.g., by Lugli *et al.* 2013) and early-memory T cells represent the sum of Tscm + Tscm-like + Tern cells. At the end of the T-cell expansion culture (approximately day 9-10), these cells may represent above 60% of total T cells.

In step c) transducing, and optionally, culturing, the immune cells comprising memory T-cells with a vector or a vector combination as described herein may be performed by methods known in the art.

For instance, a viral vector may be transduced into the immune cells by contacting them with viral particles comprising the polynucleic acid or polynucleic acid combination as described herein. Contacting may be performed at a multiplicity of infection (MOI) from 0.5 to 2, in particular of about 1, according to CAR30 expression, which corresponds to a transducing unit (TU) to T-cell ratio used of 1:2 to 2:1, in particular of about 1:1, taking into account the titer of the viral particles (TU/ml) determined as described above (e.g., by measuring CAR30$^+$ expression in Jurkat cells) T-cell comprises total of T-cells.

Contacting may optionally comprise, spinoculating the immune cells in the presence of the viral particles (e.g., at from 400 to 1200 x g, in particular from 600 to 1000 x g and yet more in particular at about $800 \times g$) for, e.g., 15 to 90 minutes, in particular from 30 to 60 minutes.

Once the cells are transduced, they are directly used for further expansion and experiments. The only cells that may be stored for subsequent use are the PBMCs before T-cell isolation (either bulk or naive).

The transduced cells obtained comprise a vector or a vector combination and a polynucleic acid or polynucleic acid combination as described herein.

Such cells are capable of expressing a protein combination as described herein, as they also comprise the first and second polynucleotide respectively encoding the CAR (a) and the modified CD200R (b) and the required genetic machinery provided by the, e.g., single vector. The genetic machinery is provided by the T cell, the part of the vector which is integrated only provides the polynucleotide sequence and the promoter to express it as a single mRNA molecule.

The transduced immune cells may further comprise the protein combination as described herein. The expression of a protein combination as described herein may be provided by the integration of the viral vector in the genome, so that the protein combination starts expressing after transduction.

For instance, the transduced immune cells may be cultured for expansion in a cell medium (e.g., complete medium such as Roswell Park Memorial Institute (RPMI) medium preferably supplemented with 10% fetal bovine serum (FBS)) in the presence of cytokines (e.g., IL-7, IL-15 and IL-21, which may be commercially available, e.g., from StemCell Technologies, Vancouver, British Columbia, Canada) to maintain a memory-enriched phenotype as previously described (Alvarez-Fernández *et al.,* 2016) The cytokines may be present at a concentration from 5 to 50 ng/ml, in particular from 10 to 40 ng/ml, more in particular of 25 ng/ml of each cytokine, such as 25 ng IL-7 + 25 ng IL-15 + 25 ng IL-21 per ml of medium. The conditions of temperature and $CO_2$ saturation may be those typically used in the art, e.g., 37 °C and 5% $CO_2$ may be suitably used.

The culture may be maintained at a target cell concentration of viable cells by replacing the cell medium including the cytokines, e.g., every three or four days. A preferred target cell concentration may be, e.g., from $5 \cdot 10^5$ to $1 \cdot 10^7$ , in particular from $1 \cdot 10^6$ to $7 \cdot 10^6$, more in particular from $2 \cdot 10^6$ to $5 \cdot 10^6$ Cells are initially seeded at a concentration $0.25 \cdot 10^6$ c/ml and at day 8-9-10 the cells may be at even $3\text{-}4 \cdot 10^6$ c/ml. The concentration of viable cells may be determined by means known in the art, e.g., by counting using the trypan blue dye exclusion assay whereby a cell suspension is mixed with the dye and then visually examined to determine whether cells take up or exclude the dye, a viable cell will have a clear cytoplasm because it will have excluded the dye, whereas a nonviable cell will have a blue cytoplasm.

The culture may be continued until the modified immune cells display a suitable expression of a protein combination as described herein Generally the culture is stopped at day 10, because at this day there are high numbers of expanded T cells and these cells still preserve an important fraction of early memory T cells (the longer the T-cell culture, the higher differentiation of the cells). For instance, the culture may run from 2 to 30 days, in particular from 3 to 20 days or from 4 to 10 days. The preferred endpoint is 10 days, because, as just mentioned the culture still preserve an important fraction of early memory T cells, e.g., at least 60 % of memory T cells with respect to the total of T-cells cells.

The expression of a protein combination as described herein may be monitored by, e.g., Flow cytometry as already indicated.

The immune cells obtained, comprising the vector or vector combination of claim, the polynucleic acid or polynucleic acid combination of any one of claims and/or the protein combination as described herein, may be referred as transduced immune cells, referring to the immune cells after transduction irrespective of whether have been subjected to expansion, or may also be referred to as modified immune cells.

**Pharmaceutical compositions, therapeutic effects, and medical treatments**

With the products and methods described herein immune cells of a patient can be modified to include a vector or vector combination, a polynucleotide or polynucleotide combination and/or a protein combination as described herein for use in the treatment of, e.g., cancer, preferably of a cancer selected from Hodgkin lymphoma, and primary mediastinal non-Hodgkin lymphoma by, e.g., returning the modified immune cells to the patient.

In particular, it has been found that such modified immune cells expressing a protein combination comprising a CD30-CAR and a modified CD200R as described herein display superior *in vitro* cytotoxicity with respect to tumor cells and cytokine secretion capacity as well as superior control of tumor growth and increase of survival *in vivo,* compared to immune cells expressing a CD30-CAR alone (without modified CD200R), even where the CD30-CAR comprised a CD28 intracellular signaling domain. Preferably the model may be a classical Hodgkin lymphoma-bearing mice.

In several embodiments modified immune cells as described herein may display an *in vitro* cytotoxicity expressed as a % of tumor cell death (also referred to herein as cell lysis) after exposing the modified immune cells to tumor cells, which is from 1.5 to 20 fold superior to immune cells expressing a CD30-CAR alone (without modified CD200R), even where the CD30-CAR comprised a CD28 intracellular signaling domain, in particular from 1.5 to 15 fold, more in particular from 1.5 to 10 fold, in particular, from 1.5 to 5 fold or from 1.5 to 4 fold, or from 1.5 to 3.5 fold. Cell lysis may be measured by methods known in the art. For instance, cell lysis may be measured after exposing the modified immune cells to tumor cells in a luciferase lysis assay as previously described in, e.g., Alvarez-Fernández et al, 2021 Tumor cells may be any suitable cells to assess a particular type of cancer, for instance cells of L-540 HL cell line may be used, which is a Hodgkin Lymphoma cell line which is commercially available from, e.g., DSMZ-German Collection of Microorganisms and Cell Cultures, Braunschweig, Germany and may be simply referred to herein as L-540 HL cells. In particular, cell lysis of the modified immune cells may be measured from 12 to 72 h, more in particular 24h after exposure to tumor cells, in particular after repetitive exposures to tumor cells (e.g., after 2, 3, 4 or 5 sequential exposures to tumor cells at 48 h intervals), at a ratio of modified immune cells to tumor cells of, e.g., 5:1 to 0.5:1, in particular from 3:1 to 1:1, and more in particular of 2.5:1. The cytotoxic capacity, expressed like cell lysis, was measured by determining the relative light units (RLU) emitted from tumor cells expressing firefly luciferase enzyme after the addition of D-luciferin. Each effector to target (E:T) ratio was compared to tumor cells without CAR30-T cells as a control. Cell lysis was calculated with the following formula:

$$\% \; cell \; lysis = 100 - (\frac{RLU \; at \; different \; E{:}T \; ratios}{Basal \; RLU \; of \; target \; cells \; alone} \cdot 100)$$

In several embodiments, modified immune cells displayed a tumor cell lysis from 20 to 98%, in particular from 25 to 95 %, more in particular from 30 to 80 %, yet more in particular from 35 to 70 % and yet more in particular from 40 to 50 % depending on the E:T ratio and the construct.

In several embodiments, modified immune cells as described herein may display a cytokine secretion which is from 1.5 to 5-fold superior to immune cells expressing a CD30-CAR alone (without modified CD200R), even where the CD30-CAR comprised a CD28 intracellular signaling domain, in particular from 1.7 to 4.7-fold, more in particular from 2 to 4.5 fold. Cytokine secretion may be measured by methods known in the art. For instance, the concentration of Th1-type cytokines (such as IFN-$\gamma$, TNF-$\alpha$ and IL-2) may be measured from cell supernatants subjected to exposure to tumor cells (e.g., L-540 HL cells) following manufacturer's instructions such as ELISA (Human IFN-$\gamma$ DuoSet ELISA; R&D Systems, Minneapolis, Minnesota, USA) or Milliplex (MILLIPLEX®; Merck, Darmstadt, Germany) In particular, cytokine secretion of the modified immune cells may be expressed as picograms (pg) of cytokine per ml of supernatant (pg/ml) and may be measured 12 to 72 h, more in particular 48 h after exposure to tumor cells, in particular after repetitive exposures to tumor cells (e.g., after 2, 3, 4 or 5 sequential exposures to tumor cells at 48 h intervals), at a ratio of modified immune cells to tumor cells of, e.g., 5:1 to 0.5:1, in particular from 3:1 to 1:1, and more in particular of 2.5:1. The amount of cytokines IFN-$\gamma$, TNF-$\alpha$ and IL-2 was determined with Graphpad Prism, which is used for fitting a standard curve and interpolating our data from this curve. In the case of Milliplex, the xPONENT software was used, which directly calculates the concentration of the cytokines In several embodiments modified immune cells as described herein may display a IFN-$\gamma$ cytokine secretion of, e.g., from 10000 to 30000 pg/ml, in particular from 12500 to 20000 pg/ml, more in particular from 15000 to 19000 pg/ml; a TNF-$\alpha$ cytokine secretion of, e.g., from 7000 to 15000 pg/ml, in particular from 8000 to 12500 pg/ml, more in particular from 9000 to 11000 pg/ml; and/or; a IL-2 cytokine secretion of, e.g., from 4500 to 10000 pg/ml, in particular from 5000 to 9000 pg/ml, more in particular from 5500 to 7500 pg/ml, measured as described herein.

The present disclosure further relates to a pharmaceutical composition comprising a protein combination, a polynucleotide or polynucleotide combination, a vector or vector combination and/or a immune cell as described herein, and a pharmaceutically acceptable ingredient.

The term "pharmaceutically acceptable ingredient", is used herein as it is commonly understood in the art. In particular, it refers to an ingredient to which physiologically tolerable and safe, so that its administration is not cause harm to an organism to which it is administered. Preferably, as used herein, the term "pharmaceutically acceptable" means that it has been approved by a federal or state government regulatory agency or that it is listed in the US Pharmacopeia, the European Pharmacopeia (Ph. Eur.) or other generally recognized pharmacopoeia for use in animals and, in particular, in humans.

Pharmaceutically acceptable ingredients suitable for pharmaceutical compositions as described herein may include a pharmaceutically acceptable carrier, a diluent, and/or an excipient.

For instance, a pharmaceutically acceptable ingredient may be selected from water, saline, dextrose, glycerol, and combinations thereof.

The present invention further relates to a protein combination, a polynucleotide or polynucleotide combination, a vector or vector combination, an immune cell or a pharmaceutical composition as described herein, for use in a medical treatment, preferably in the treatment of cancer, and more preferably in the treatment of a cancer selected from Hodgkin lymphoma, and primary mediastinal non-Hodgkin lymphoma.

"Treatment" refers to the medical management of a disease, disorder, or condition of a subject (as defined above) for the maintenance or amelioration of the health of the subject or to prophylactic treatments for preventing the degradation of health in a subject. In other words, to elicit a therapeutic or prophylactic benefit to the subject.

A protein combination, a polynucleotide or polynucleotide combination, a vector or vector combination, an immune cell and a pharmaceutical as described herein, may also be suitably used in the preparation of a medicament for the treatment of cancer, preferably of a cancer selected from Hodgkin lymphoma, and primary mediastinal non-Hodgkin lymphoma.

A protein combination, a polynucleotide or polynucleotide combination, a vector or vector combination, an immune cell or a pharmaceutical composition, for use in a treatment as described herein may be administered in a manner appropriate to the disease or condition to be treated (or prevented) as determined by persons skilled in the medical art. An appropriate dose, suitable duration, and frequency of administration of the compositions will be determined by such factors as the condition of the patient, size, type, stage and severity of the disease, particular form of the active ingredient, and the method of administration.

For instance, a medical treatment may comprise

a) manufacturing immune cells ex *vivo* / *in vivo* from a biological sample previously isolated from a subject as described herein to provide modified immune cells comprising a protein combination, a polynucleotide or polynucleotide combination and/or a vector or vector combination, as described herein, and
b) administering the modified immune cells to the same subject.

Administering may be performed at a "therapeutically effective amount" or at a "therapeutically effective administration regimen" of modified immune cells, in the context of a disease or condition being treated. These expressions refer to a number of cells or a dosage regime of such cells that result in a therapeutic or prophylactic/preventive benefit in a statistically significant manner, including an improved clinical outcome; lessening, alleviation or amelioration of one or more of the symptoms associated with a disease being treated; decreased occurrence of symptoms; improved quality of life; longer disease-free status; diminishment of extent of disease, stabilization of disease state; delay of disease progression; remission; survival; prolonged survival; or any combination thereof. In particular, the therapeutic or prophylactic/preventive benefit may include, reducing tumor size, inhibiting cancer growth or the like.

In this text, the term "includes", "comprises" and derivations thereof (such as "including", "comprising", etc.) should not be understood in an excluding sense, that is, these terms should not be interpreted as excluding the possibility that what is described and defined may include further elements, steps, etc.

On the other hand, the disclosure is obviously not limited to the specific embodiment(s) described herein, but also encompasses any variations that may be considered by any person skilled in the art (for example, as regards the choice of materials, dimensions, components, configuration, etc.), within the general scope of the invention as defined in the claims.

The present invention is further illustrated by the following examples without being limited thereto or thereby.

**EXAMPLES**

**Example 1** - **Gene synthesis and cloning of CAR (a) and modified CD200R (b) constructs**

A construct for the expression of a protein combination comprising a CD30-CAR and a modified CD200R according to the invention, and comparative examples comprising a CD30-CAR and without modified CD200R, were synthesized and subcloned into a replication-incompetent third-generation lentiviral vector backbone (pHIV7) to generate the transfer vectors for later use in lentiviral production as follows.

Example 1 and Comparative Example 1

Firstly, the CD30-CAR construct was assembled by fusing polynucleotides encoding different protein domains:

- a CD8α signal peptide consisting of the amino acid sequence of SEQ ID No. **18,** which helps to direct the protein combination of the invention to the cell membrane and is later released and it is not part of the functional protein combination,

i) an ectodomain comprising a

- a CD30-binding domain consisting of a scFv comprising the variable regions of heavy and light chains of a monoclonal antibody (mAb) specifically targeted against CD30 antigen (mAb; clone T105 comercial) (Nagata *et al.* 2005), which heavy and light chains of the mAb were separated by a "Whitlow" peptide linker (Whitlow *et al.* 1993) consisting of the amino acid sequence of SEQ ID **No. 2,**
- a CD8α hinge domain consisting of the amino acid sequence of SEQ ID No. **19;**

iii) a transmembrane domain of CD8α consisting of the amino acid sequence of SEQ ID No. **16;** and
ii) an endodomain comprising a 4-1BB-CD3ζ signaling module consisting of the amino acid sequence of SEQ ID No. **15.**

The polynucleic acid molecule encoding the CD30-CAR comprised the following elements (5' to 3'): the CD8α signal peptide; the ectodomain (i) comprising the CD30 binding domain, and the CD8α hinge domain; the CD8α transmembrane domain (iii) and the endodomain (ii) comprising a 4-1BB-CD3ζ signaling module.
The polynucleotide sequence encoding the whole CD30-CAR corresponded to SEQ ID No. **22,** and the sequence of the corresponding CD30-CAR corresponded to the amino acid sequence of SEQ ID No. **21** and, once the signal peptide is released, to residues 22-498 of the amino acid sequence of SEQ ID No. **21.**
Secondly, a fusion CD200R construct was assembled by fusing polynucleotides encoding different protein domains as previously described by Oda *et al.* 2017, and international patent applications published as WO 2016/141357 and WO 2018/170475: For Example 1 the encoded protein domains for the modified CD200R comprised:

iv) an extracellular domain comprising a

- binding domain of CD200R consisting of the amino acid sequence of SEQ ID No. **23,** and
- an extracellular portion of CD28 connecting the binding domain of CD200R and the transmembrane domain, the extracellular portion of CD28 consisting of 12 amino acids of the amino acid sequence of SEQ ID No. **27** corresponding to amino acids 141-152 of the wild-type sequence SEQ ID NO. **13.**

v) a transmembrane domain of CD28 corresponding to the 153-179 amino acids from the N-terminus of wild-type human CD28 P10747; UNIPROT; version 221 released on 08-Nov-2023, consisting of the amino acid sequence of SEQ ID No. **17;**
vi) an intracellular stimulatory domain (vi) of CD28, corresponding to amino acids 180-220 of wild-type human CD28 P10747; UNIPROT; version 221 released on 08-Nov-2023, the intracellular domain (iv) consisting of the amino acid sequence of SEQ ID No. **28.**

The polynucleic acid molecule encoding the CD200R-CD28 fusion protein comprised the following elements (5' to 3'): the extracellular domain comprising a CD200R binding domain followed by the extracellular portion of CD28; the CD28 transmembrane domain and the intracellular stimulatory domain of CD28.
The polynucleotide sequence encoding the whole fusion CD200R-CD28 protein corresponded to SEQ ID No. **30,** and the sequence of the corresponding fusion CD200R-CD28 protein corresponded to the amino acid sequence of SEQ ID No. **29.**
For Comparative example 1 instead of a fusion CD200R protein (such as CD200R-CD28 of example 1) a truncated fusion protein (CD200R28t) was used. The encoded protein domains for the truncated CD200R (CD200Rt) fusion protein comprised:

- an extracellular domain (iv) of CD200R and CD28 as defined for Example 1
- a transmembrane domain (v) of CD28 as defined for Example 1 and

- no intracellular domain (iv).

The polynucleotide sequence encoding the whole truncated fusion CD200R protein (CD200Rt) corresponded to SEQ ID No. **33,** and the sequence of the corresponding truncated fusion CD200R protein corresponded to the amino acid sequence of SEQ ID No. **34.**

The CD30-CAR construct was joined in *cis* to region encoding a T2A ribosomal "skipping" peptide, followed by a chimeric co-stimulatory CD200R, in a single polynucleic acid molecule comprising the following elements (5' to 3'): CD30-CAR construct; T2A encoding region; modified CD200R construct.

The nucleotide sequence of the single nucleic acid molecule for Example 1, may be referred to as CD30CAR_CD200R-CD28 or CAR30_CD200R28 construct and corresponded to SEQ ID No. **31.** The protein sequence expressed by the nucleotide sequence of Example 1, may be referred to as CD30CAR_CD200R-CD28 or CAR30-CD200R28 and corresponded to SEQ ID No. **32.**

The nucleotide sequence of the single nucleic acid molecule for Comparative Example 1, may be referred to as CD30CAR_trunc-CD200R-CD28 or CAR30_CD200R28t construct and corresponded to SEQ ID No. **35.** The protein sequence encoded by the nucleotide sequence of Comparative Example 1, may be referred to as CD30CAR_trunc-CD200R-CD28 or CAR30-CD200R28t and corresponded to SEQ ID No. **36.**

As indicated above, the single polynucleic acid molecule was synthesized and subcloned into a replication-incompetent third-generation lentiviral vector backbone (pHIV7) to generate the transfer vectors for later use in lentiviral production.

Comparative Examples 2a and 2b

Transfer vectors for lentiviral production were prepared according to Example 1, for a CD30-CAR in absence of the modified CD200R construct (comparative example 2a) and for a CD30-CAR incorporating an intracellular CD28 signaling domain also in absence of the modified CD200R construct (comparative example 2b).

The CD30-CAR construct of Comparative example 2a corresponded to the CD-30 construct of Example 1 and Comparative Example 1 above, with the same amino acid sequence (of SEQ ID No. **21)** and the same polynucleotide sequence encoding the CD30-CAR (SEQ ID No. **22).**

The polynucleic acid molecule of Comparative example 2b encoding the CD30-CAR incorporating an intracellular signaling domain of CD28 (also referred to as CD30CAR_CD28 construct or CAR30_CD28) comprised the following elements (5' to 3'): the CD8α signal peptide; the ectodomain (i) comprising the CD30 binding domain, and the CD8α hinge domain; the CD8α transmembrane domain (iii) and the endodomain (ii) comprising a CD28 signaling domain followed by a 4-1BB-CD3ζ signaling module. However, the CD8α signal peptide the signal peptide was released and was not part of the functional protein, as explained above.

The polynucleotide sequence encoding the CAR30_CD28 construct of comparative example 2b corresponded to SEQ ID No. **37,** and the sequence of the encoded CD30CAR_CD28 protein corresponded to the amino acid sequence of SEQ ID No. **38.**

In order to preserve a similar vector construct structure and size the modified CD200R construct was substituted by a sequence encoding a truncated Epidermal Growth Factor Receptor (EGFRt) which was used as a reporter gene. The polynucleotide sequence encoding the EGFRt corresponded to SEQ ID No. **39,** and the sequence of the encoded EGFRt protein corresponded to the amino acid sequence of SEQ ID No. **40.**

The nucleotide sequence of the single nucleic acid molecule for Comparative example 2a, may be referred to as CD30CAR_EGFRt or CAR30_EGFRt construct and corresponded to SEQ ID No. **41.** The amino acid sequence encoded by the nucleotide sequence of Comparative example 2b, may be referred to as CD30CAR_EGFRt or CAR30_EGFRt construct and corresponded to SEQ ID No. **42.**

The nucleotide sequence of the single nucleic acid molecule for Comparative example 2b, may be referred to as CD30CAR-CD28_EGFRt or CAR30-CD28_EGFRt construct and corresponded to SEQ ID No. **43.** The amino acid sequence encoded by the nucleotide sequence of Comparative example 2b, may be referred to as CD30CAR_EGFRt or CAR30_EGFRt construct and corresponded to SEQ ID No. **44.**

A summary of the different elements of the constructs of Examples 1a and 1b and of comparative examples 1a and 1b can be found on the following table (Table **1).**

| | Example | Comparative Examples | | |
|---|---|---|---|---|
| | 1 | 1 | 2a | 2b |
| Construct and vector name | CAR30_ CD200R28 | CAR30_trunc-CD200R28 | CAR30_ EGFRt | CAR30-CD28_EGFRt |

(continued)

| | Example | Comparative Examples | | |
|---|---|---|---|---|
| | 1 | 1 | 2a | 2b |
| Polynucleotide SEQ ID No. | *31* | *35* | *41* | *43* |
| Amino acid SEQ ID No. | *32* | *36* | *42* | *4744* |
| **Domains encoded by the transfer vectors** | | | | |
| **CAR30** | | | | |
| CD8α signal peptide | YES | YES | YES | YES |
| CD30-binidng domain | YES | YES | YES | YES |
| CD8α hinge domain | YES | YES | YES | YES |
| CD8α transmembrane domain | YES | YES | YES | YES |
| 4-1BB-CD3ζ signaling module | YES | YES | YES | NO |
| CD28-4-1BB-CD3ζ signaling module | NO | NO | NO | YES |
| **2A peptide** | | | | |
| T2A | YES | YES | YES | YES |
| **Modified CD200R** | | | | |
| CD200R binding domain | YES | YES | NO | NO |
| CD28 extracellular portion | YES | YES | NO | NO |
| CD28 transmembrane domain | YES | YES | NO | NO |
| CD28 intracellular domain | YES | NO | NO | NO |
| **EGFRt in substitution of CD200R** | | | | |
| EGFRt | NO | NO | YES | YES |

A schematic representation of protein expressing domains and regulatory domains in polynucleotide constructs can be found in Figure 1. Each construct represented respectively expressing A) a protein combination comprising a CAR (a) directed to CD30 and a modified CD200R (b) according to Example 1 (CAR30_CD200R28); B) a protein combination comprising a CAR (a) according to Comparative Example 2a (CAR30_ EGFRt); and C) a protein combination comprising a CAR (a) directed to CD30 additionally comprising a CD28 intracellular signaling domain according to Comparative Example 2b (CAR30-CD28_ EGFRt).

A schematic representation of the proteins expressed can be found in Figure 2, wherein A) represents a protein combination comprising a CAR (a) and a modified CD200R (b) according to Example 1 (CAR30_CD200R28); B) represents a CAR (a) according to Comparative Example 2a (CAR30); C) represents a CAR (a) comprising a CD28 intracellular signaling domain according to Comparative Example 2b (CAR30-CD28).

## Example 2 - Lentiviral production

Lenti-X™ 293 T-cells (from Clontech Laboratories, Inc., Mountain View, California, USA) were independently co-transfected with one of the transfer lentiviral vectors of Example 1 (CD30CAR_CD200R-CD28), Comparative Example 1 (CD30CAR_trunc-CD200R-CD28), comparative example 2a (CD30CAR_EGFRt) and comparative example 2b (CD30CAR-CD28_EGFRt), and

- two packaging vectors (pCHGP-2 codifying for gag and pol genes; and pCMV-rev2 codifying for rev gene) and
- one envelope vector (pCMV-VSV-G codifying for the Vesicular Stomatitis Virus envelope Glycoprotein)

The packaging and envelope vectors were from Addgene, Watertown, Massachusetts, USA.

Transfer, packaging, and envelope vectors were previously amplified in competent *Escherichia coli* (Stbl3™; Thermo Fisher, Germany). The competent *E. coli* were heat-shock transformed with the vectors of Example 1 and grown at 37°C for 16-18 hours at 220-240 rpm. Plasmid purification was using by HiSpeed® Plasmid Maxi Kit (Qiagen, Germany), according to manufacturer's instructions.

Co-transfection of the purified transfer, packaging, and envelope vectors was performed using with a calcium solution and HEPES-Buffered Saline (CalPhos™ Mammalian Transfection Kit; Clontech Laboratories, Inc., Mountain View, California, USA), following the manufacturer instructions. For transfection, DNA needs to be mixed with $CaCl_2$ solution and then drop-wise added to the HEPES-Buffered Saline, which helps to optimally introduce the genetic material into Lenti-X cells, Chen *et al.* 2018 (Chen et al., Utilization of HEPES for Enhancing Protein Transfection into Mammalian Cells., Mol Ther. Methods Clin. Dev. 2018;13:99-111, doi:10.1016/j.omtm.2018.12.005).

Culture medium was changed 16 hours after transfection and the cells were incubated for another 36-48 hours, time when lentiviral supernatants were collected. Firstly, lentiviral supernatants were centrifuged to remove cellular debris for 15 minutes at 3600 rpm and then ultracentrifuged to concentrate lentiviral particles for 2 hours at 24500 rpm (4-8°C). The lentiviral pellet was resuspended in TBS-5 buffer, snap-frozen on dry ice and stored at -80°C to provide frozen lentiviral particles. The lentiviral particles are frozen until use, in order to increase the half-life of the stock.

### Example 3 - Lentiviral titration

Lentiviral titration was performed in Jurkat cells (from DSMZ-German Collection of Microorganisms and Cell Cultures, Braunschweig, Germany). Tumor cells (L540 cells which are a Hodgkin lymphoma cell line) Jurkat cells are used for lentiviral titration in the field. L540 cells are a Hodgkin lymphoma cell line that is used for experimental analyses with CAR30-T cells. Jurkat cells were transduced with different volumes of thawed lentiviral particles obtained in Example 2 (e.g.: 0,25 µl, 0,5 µl, 1 µl, 2,5 µl...) in the presence of polybrene at 5 µg/ml (Sigma-Aldrich, St. Louis, Missouri, USA) and incubated for 48 hours.

After this time, Jurkat cells were stained with CD30 recombinant protein conjugated to a fluorochrome to be further analyzed by flow cytometry to quantify the fraction of cells expressing CD30-CAR on the cell membrane (also referred to as CAR30+ cells) for each volume of lentiviral particles by flow cytometry.

Lentiviral titer was expressed in Transducing Units per milliliter (TU/ml) after using the following formula:

$$TU/ml = \frac{(Total\ number\ of\ Jurkat\ cells) \cdot (\%\ CAR30^+\ cells)}{(Volume\ of\ lentivirus\ that\ transduces\ aproximately\ 50\%\ of\ Jurkat\ cells)}$$

### Example 4 - Generation of memory-enriched T-cells comprising CD30-CAR and modified CD200R

Memory-enriched T-cells comprising CD30-CAR and, if applicable also, modified CD200R also referred to as CAR30+ memory-enriched T-cells or CAR30+ and CD200R+ memory-enriched T-cells (or simply referred to jointly as CAR30 T-cells) were obtained as follows:

CD4+ and CD8+ bulk T-cells were isolated from thawed peripheral blood mononuclear cells (PBMCs) obtained from five independent healthy donors, using the EasySep™ Human T-cell Isolation Kit (StemCell Technologies, Vancouver, British Columbia, Canada), according to the manufacturer's instructions.

The isolated T-cells were seeded at day 0 at a concentration of $2.5 \cdot 10^5$ cells/ml per well in a 24-well plate. The T-cells were activated during the first 48 hours with α-CD3/CD28 magnetic beads (Dynabeads™; Life Technologies, Waltham, Massachusetts, USA) in a 1:2 bead to T-cell ratio as previously described (Alvarez-Fernández *et al.,* 2016). At day 2, magnetic beads were removed from culture previous to T-cell transduction.

Using the lentiviral particles obtained according to Example 2 for each one of the constructs of Example 1 and Comparative Example 1 and Comparative Examples 2a and 2b, five T-cell transductions (one per each healthy donor) were performed at a multiplicity of infection (MOI) of 1 (that is a 1:1 transducing unit (TU) to T-cell ratio according to CAR30+ expression in Jurkat cells (TU/ml) as determined in Example 3).

Two-thirds of cell medium were removed from each well to favor viral adsorption and then cells were spinoculated at 800 x *g* (800 units of gravity) for 45 minutes at 32°C in the presence of polybrene at 5 µg/ml (Sigma-Aldrich, St. Louis, Missouri, USA). Cell medium was added to a final volume of 1 ml after 4 hours.

T-cells were cultured for expansion in the presence of IL-7, IL-15 and IL-21 (StemCell Technologies, Vancouver, British Columbia, Canada) at a concentration of 25 ng/ml to maintain a memory-enriched phenotype (Alvarez-Fernández *et al.* 2016).Cell medium and cytokines were replaced every three or four days, when cells were also counted by trypan blue dye exclusion and maintained at a concentration of $1 \cdot 10^6$ cells/ml.

T-cells were cultured in complete medium, which consisted of Roswell Park Memorial Institute (RPMI) Biowest, France 1640 with L- glutamine supplemented with 10% fetal bovine serum (FBS) (ThermoFisher, Germany) and were maintained at 37°C in 5% $CO_2$. The culture was continued for ten (10) days, when T-cell expansion culture was ended obtaining CAR30-T cell products which are analyzed in vitro and in vivo for functional performance.

A schematic diagram of the procedure can be found in figure 3. The expression of CAR30, CD200R, EGFRt in the transduced T-cells was analyzed by flow cytometry at days 6 and 10 of T-cell culture. Relevant differences in CD200R intensity of expression were seen when comparing different CAR30-T cells along T-cell culture. CAR30 and CAR30CD28 -T cells showed CD200R only at a low intensity of expression (CD200Rlow), while CAR30-CD200R28-T cells showed CD200R both at a low and at a high intensity of expression (CD200Rhigh) in cell membrane. Since CD200Rhigh T cells, but not CD200R-/low, were described to be CAR30+, low was associated to endogenous expression and high was associated to the sum of endogenous and transgenic expression of CD200R. At day 10 there was a co-relation between CAR30 expression and either CD200Rhigh or EGFRt in CAR30-CD200R28 and both CAR30 and CAR30CD28-T cells, respectively.

## Example 5 - In vitro performance of CAR30 T-cells

### Example 5a -Continuous re-exposure of CAR30-T-cells to tumor assay *in vitro*

CAR30[+]_EGFRt [+] T-cells and CAR30[+]_modified CD200R[+] T-cells, jointly referred to herein as transduced T-cells or CAR30 T-cells, serial co-cultures with L-540 HL cell line (CD30[+] CD200-L[+]), obtained from DSMZ-German Collection of Microorganisms and Cell Cultures, Braunschweig, Germany, were performed to study the antitumor capacity of the different constructs (of examples 1a and 1b and of comparative examples 1a and 1b). L-540 cell line was genetically modified in our laboratory by using a lentiviral vector to express enhanced green fluorescence protein (eGFP) and firefly luciferase (ffluc) (Addgene, Watertown, Massachusetts, USA). GFP was used for fluorescence-activated cell sorting of transduced L-540 cells expressing ffluc. Ffluc+ L-540 cells were used for in vitro cytotoxicity assays and for in vivo detection and sizing of the tumor.

For each construct tested, $5 \cdot 10^5$ transduced T-cells/ml per well were cultured in a 24-well plate, in a cytokine-free medium using the same medium than T-cell expansion culture but without the addition of any cytokine in the presence of tumor cells (from day 10 on) and re-exposed to tumor every 48 hours in a 2.5:1 effector to target ratio (E:T ratio), considering only EGFRt[+] or CD200Rhigh T-cells as effector cells and the target being the tumor cells (i.e., L-540 HL cell line). The expression of CD200R and EGFRt was quantified? by flow cytometry: effector cells mean transduced T cells expressing either EGFRt+ or CD200R at high intensity.

The CAR30 T-cells were subjected to a series of exposures to tumor, having a total of 5 rounds of tumor exposure.

In each series of tumor exposure to tumor, the same volume of cytokine-free fresh medium was added within the different CAR30 T-cell co-cultures (i.e., for each of examples 1a and 1b and comparative examples 1a and 1b)

Before tumor exposure (i.e., at the end of T-cell expansion culture (day 10)) and 48 h after each round of tumor exposure cytotoxicity assays and cytokine secretion assessments (IFN-γ, IL-2, TNF-α) were performed as detailed below and in the following examples 5b and 5c.

A schematic diagram of the procedure can be found in figure 4.

Cytotoxicity assays at different effector to target ratios were performed at the end of T-cell expansion culture (day 10) of example 4, at 48 hours after the first and each subsequent tumor exposure. CAR30-T cell cytotoxicity was evaluated at day 10 (which corresponds with the end of the T-cell expansion culture) and 48 hours after each exposition to the tumor (being the first exposition to the tumor at day 10 of the T-cell expansion culture). The methods and results of the cytotoxicity assays are explained in Example 5b below and summarized in the corresponding graphs of figure 5.

For cytokine secretion assessment (IFN-γ, IL-2, TNF-α 48 hours after of tumor exposure, the co-cultures had their cell medium completely renovated by centrifugation so just the cytokines secreted after each exposure to tumor could be measured and not the remaining cytokines of previous exposures. The methods and results of the cytokine secretion assessment are explained in Example 5c below and compiled in the corresponding graphs of Figure 6 and Figure 8.

### Example 5b - Cytotoxicity assays

*In vitro* short-term cytotoxicity of CAR30 T-cells (transduced with the constructs of examples 1a & 1b and comparative examples 1a & 1b) and un-transduced T-cells were evaluated for their cytotoxic capacity against HL cell line L-540 cells (CD30[+] CD200-L[+]), also referred herein as tumor cells.

Cytotoxicity assays were performed at the end of T-cell expansion culture (day 10), (of example 4, before exposure to tumor cells) at 24 hours after the first exposure to tumor cells and at 48 hours after the first and each subsequent exposure to tumor cells (of example 5). The cytotoxicity was evaluated at 24 hours (and also at 48 hours for the first tumor exposure). Meanwhile, tumor exposures (co-cultures) were performed every 48 hours.

Cytotoxicity was evaluated by taking samples of the CAR30 T-cells before being exposed to tumor cells at the indicated time intervals.

CAR30 T cells at day 10 and 48 hours after being exposed at a 2.5:1 E:T ratio were used in a luciferase cell lysis assay (where the cells were exposed at different E:T ratios starting from 20:1 to 0.15:1)

Tumor cells where then subjected to different effector to target ratios (E:T) of the CAR30 T-cells (considering only EGFRt+ or CD200R$^{high}$ as effector T-cells as determined according to Example **5** and tumor cells as the target cells). The tested E:T ratios were: 20:1, 10:1, 5:1, 2.5:1, 1.25:1, 0.6:1, 0.3:1 and 0.15:1. After 24 hours of exposure the percentage (%) of cell lysis for each E:T ratio was determined.

CAR-T cells were co-cultured in triplicate with ffluc+ L-540 cells at different E:T ratios (20:1; 10:1; 5:1; 2,5:1; 1,25:1; 0,6:1; 0,3:1; 0,15:1) at 37°C for 48 hours. Every 24 hours 2 μl of D-luciferin potassium salt bioluminescent substrate (XenoLight™; Perkin Elmer, Waltham, Massachusetts, USA) were added in each well from a stock solution at 15 mg/ml and incubated at 37°C for 20 minutes. Then, bioluminescence data were measured as relative light units (RLU) using the Infinite M200 PRO Plate Reader (Tecan, Switzerland).

Triplicate wells were averaged, and tumor-cell lysis was calculated from data with the following equation:

$$\% \ cell \ lysis = 100 - [(RLU \ at \ different \ E{:}T \ ratios \ / \ Basal \ RLU \ of \ target \ cells \ alone) \cdot 100]$$

Statistical analyses were performed with GraphPad Prism 6 (GraphPad Software, USA). Data are shown as the mean $\pm$ SEM from five independent measurements of CAR30 T-cells obtained from five different healthy donors. Differences were tested for statistical significance using one-way ANOVA test. A p value <0.05 was considered significant.

The cytotoxity assay protocol was adapted from the following publication Alvarez-Fernández *et al.* 2021,

The co-culture is performed every 48 hours. We take the $5\cdot10^5$ transduced cells from every condition of CAR30-T cells were taken and we exposed them again to tumor cells in a ratio E:T 2.5:1 ($2\cdot10^5$ L540 cells) (total volume of 1 ml). From those wells, every 48 hours: cells are counted and, one part of them is used in the luciferase cell lysis assay, while another part is used for the next exposition to tumor cells (E:T 2.5:1).

As an example, in order to seed $5\cdot10^5$ transduced cells per well, 0.6 ml were used for CAR30_EGFRt (Comparative Example 2a), 0.7 ml for CAR30-CD28_EGFRt (Comparative Example 2b), and 0.65 ml for CAR30_CD200R28 (Example 1). Those wells containing less than 0.7 ml of volume, an extra of 0.1 ml and 0.05 ml, respectively, of supernatants were added so all of the well would have 0.7 ml of volume with the same number, $5\cdot10^5$, of transduced cells. Subsequently, 0.3 ml of cytokine-free fresh media (with $2\cdot10^5$ L-540 cells) would be equally added to the three CAR30-T cell cultures. This was done to ensure that all the three CAR30-T cells are cultured in the same conditions of naturally-secreted cytokines.

The plot against the E:T ratios for each cytotoxicity assay for the CAR30 T-cells of each construct of Example 1 (CAR30_CD200R28 triangles) and Comparative Examples 2a (CAR30_EGFRt diamonds) and 2b (CAR30CD28_EGFRt squares) is shown in Figure 5, at 24 hours after a first exposure (A), at 48 hours after a first exposure (B), and at 48 hours after a second exposure (C), a third (D), a fourth (E) and a fifth (F) exposure.

Looking at the results of cytotoxicity for the indicated number of exposures and time after the exposures, the following results can be compared from the measurement of cytotoxicity different effector to target ratios. Looking at the measurement 24h after exposure at an E:T ratio of 2.5:1 for each CAR30 T-cell pre-exposed to the tumor cells.

24 hours after a first tumor exposure at a 2.5:1 E:T ratio (effector to target ratio, considering only EGFRt+ or CD200R$^{high}$ as effector T-cells as determined according to Example 5), no significant differences in killing capacity between the constructs were seen. The recorded tumor cell death at 2.5:1 E:T ratio after 24 hours was 9 $\pm$ 6,1% for T-cells with constructs of Example 1 (CAR30_CD200R28), *versus* 27,2 $\pm$ 14,6% for Comparative example 2a (CAR30_EGFRt) and 18,8 $\pm$ 11,9% for Comparative Example 2b (CAR30-CD28_EGFRt) a level of significance of p=0,6082 (Figure **5** A). Therefore, the difference was not of statistical significance.

The recorded tumor cell death at 2.5:1 E:T ratio after 48 hours of the first exposure, for a cytotoxicity measured at 2.5:1 E:T ratio was 96.2 t 1.4% for Example 1 (CAR30_CD200R28), *versus* 94.2 $\pm$ 2.7% for Comparative Example 2a (CAR30_EGFRt), and 95.2 $\pm$ 2% for Comparative Example 2b (CAR30-CD28_EGFRt) with a level of significance of p=0.8558 (Figure **5** B). Therefore, the difference was not of statistical significance.

Subsequently, measurements were taken when the CAR30 T-cells were put under stress conditions, similar to those that they may face in tumor microenvironment *in vivo,* by re-exposing the CAR30 T-cells several times to tumor cells, i.e., to L-540 HL cells every 48 hours at a 2.5:1 effector to target ratio, considering only EGFRt+ or CD200R$^{high}$ as effector T-cells, since it is the highest tumor burden that all CAR30 T-cells can kill after 48 hours. This way, CAR30-T-cells were continuously stimulated with antigen, with very short rest times between tumor exposures. The highest tumor burden refers to the maximum number of tumor cells that a certain number of CAR30-T cells can kill in a period of time of 48 hours (that is: in 48 hours CAR30-T cells can kill all L-540 cells when they are 2,5 times more frequent than tumor cells).

After the fifth exposure to tumor cells CAR30_CD200R28 T-cells (of Example 1) showed a significantly higher cytotoxic activity compared to CAR30_EGFRt T-cells (Comparative Example 2a) and to CAR30CD28_ EGFRt (comparative example 2b) with a tumor cell death at a 1.25:1 E:T ratio after 24 hours of 47.8 $\pm$ 17% for Example 1 (CAR30_CD200R28) *versus* 4.6 $\pm$ 4.4% for comparative example 2a (CAR30_ EGFRt) and 15.6 $\pm$ 10.1% for comparative example 2b (CAR30-CD28_ EGFRt) with a statistical significance of p<0.0001 and p=0.0002, respectively (Figure 5F). Therefore, in this case the difference was of statistical significance.

In these experiments CAR30_CD200R28 T-cells of Example 1 showed superior *in vitro* killing capacity after serial tumor exposures compared to CAR30 T-cells without modified CD200R (comparative example 2a), and even where the CAR30 comprised a CD28 intracellular signaling domain (comparative example 2b).

Example 5c - Cytokine-secretion assessment

To further study functional performance of CAR30 T-cells, the main Th1-type cytokines (IFN-$\gamma$, TNF-$\alpha$ and IL-2) were analyzed from cell supernatants after 48 hours of repeated CAR30-T-cell exposures to tumor cells, as described in Example 5, at a 2.5:1 effector to target ratio (EGFRt$^+$ or CD200R$^{high}$ Tcells to L-540 HL cells).

The cell medium was completely renovated by centrifugation 48 hours before the collection of cell supernatants so just the cytokines secreted after the specifically studied exposure to tumor could be measured and not the remaining cytokines of previous exposures.

Cytokine-secretion was measured by determining the amount of Th1-type cytokines (IFN-$\gamma$, TNF-$\alpha$ and IL-2) The specified suppliers above have their own protocol, which was followed. As previously described: IL-2 and TNF-$\alpha$ detection was performed by a multiplex assay (MILLIPLEX®; Merck, Darmstadt, Germany) using Luminex® Technology. IFN-$\gamma$ detection was done by sandwich Enzyme-Linked ImmunoSorbent Assay (ELISA) (Human IFN-$\gamma$ DuoSet ELISA; R&D Systems, Minneapolis, Minnesota, USA).

In Figure 6 the results are shown of the measurements of (A) IFN-$\gamma$, (B) TNF-$\alpha$ and (C) IL-2 for the different types of CAR30 T-cells tested of Example 1 (CAR30_CD200R28 T-cells, light grey bars), comparative example 2a (CAR30_EGFRt T-cells, black bars) and comparative example 2b (CAR30-CD28_EGFRt dark gray bars), after 1, 2, 3 and 4 exposures to tumour.

In Figure 8 the results are shown for measurements after one exposure of (A) IFN-$\gamma$, (B) TNF-$\alpha$ and (C) IL-2 for the different types of CAR30 T-cells tested of Example 1 (CAR30_CD200R28 T-cells, light grey bars), comparative example 2a (CAR30_EGFRt T-cells, black bars) and comparative example 2b (CAR30-CD28_EGFRt dark gray bars), and comparative example 2 (CAR30_CD200R28t T-cells, white bars), after 1 exposure to tumour.

Statistical analyses were performed with GraphPad Prism 6 (GraphPad Software, USA). Data are shown as the mean $\pm$ SEM from four independent measurements of CAR30 T-cells obtained from four different healthy donors. Differences were tested for statistical significance using one-way ANOVA test. A p value <0.05 was considered significant. In the graphs of Figures 6 and 8 * means p < 0.05; ** means p < 0.01; and *** means p < 0.001, indicating statistically significant differences.

CAR30_CD200R28 T-cells (of Example 1) showed increased cytokine secretion after tumor encounter. The results of cytokine secretion assays are presented in Figure 6, wherein the data represent mean $\pm$ SEM of four independent healthy donors and * means p<0,05.

The measured **IFN-$\gamma$** secretion was 17753.6 $\pm$ 4621.4 pg/ml for CAR30_CD200R28 *versus* 8597.1 $\pm$ 2027.9 pg/ml for CAR30_ EGFRt and 5680.4 $\pm$ 1069.6 pg/ml in CAR30-CD28_ EGFRt; with a significance of p=0.0432 and p=0.0436, respectively (Figure 6 A). Therefore, the difference in cytokine secretion was of statistical significance.

The measured **TNF-$\alpha$** secretion was 10268.6 $\pm$ 1656.4 pg/ml for CAR30_CD200R28 *versus* 4935.2 $\pm$ 464.8 pg/ml for CAR30_ EGFRt and 5705.1 $\pm$ 444.9 pg/ml for CAR30-CD28_ EGFRt; with a significance of p=0.0276 and p=0.0547, respectively) (Figure **6** B). Therefore, the difference in cytokine secretion was of statistical significance.

The measured **IL-2** secretion was of 6161.2 $\pm$ 1588.8 pg/ml for CAR30_CD200R28 *versus* 1359,1 $\pm$ 341 pg/ml CAR30_ EGFRt (of Comparative Example 2a) and of 3548.5 $\pm$ 801.5 pg/ml in CAR30-CD28_ EGFRt (of Comparative Example 2b); with a significance of p=0.0349 and p=0.1341, respectively (Figure **6** C). Therefore, the difference in cytokine secretion was of statistical significance.

Superior cytokine secretion capacity of CAR30_CD200R28 T-cells (of Example 1) was maintained (when compared to the Comparative Examples 2a and 2b) after every subsequent re-exposure to tumor cells.

Further, after the first tumor exposure IL-2 secretion was also found to be higher in CAR30-CD28_EGFRt T-cells (of Comparative Example 2b) compared to CAR30_EGFRt T-cells (of Comparative Example 2a) with a significance of p=0,0296 (Figure **6** C). Therefore, in this case the difference in cytokine secretion was also of statistical significance as for TNFa and IFNg.

As it can be seen from Figure 8, CAR30-trunc-CD200RCD28-T cells (of Comparative Example 1) showed a significantly lower secretion of these cytokines compared to CAR30_CD200R28 (of Example 1). IFN-$\gamma$ secretion was 1992.7 $\pm$ 426.3 pg/ml for Comparative Example 1 vs 6278.3 $\pm$ 915.7 pg/ml for Example 1; p=0,0051) (Figure 8A). TNF-$\alpha$ secretion was 796 $\pm$ 165 pg/ml for Comparative Example 1 vs 5524.6 $\pm$ 801.4 pg/ml for Example1; p=0.0013) (Figure 8B). IL-2 secretion was 140.8 $\pm$ 73.2 pg/ml for Comparative Example 1 vs 12120.5 $\pm$ 1772.5 pg/ml for Example 1; p=0.0005) (Figure 8C). Moreover, for Comparaative Example 1 cytokine-secretion capacity was comparable to those of Comparative Examples 2a and 2b (IFN-$\gamma$: p=0,376 and p=0,2912, CAR30BBz-CD200Rt vs CAR30BBz and CAR3028BBz-T cells) (TNF-$\alpha$: p=0,0879 and p=0,0719, CAR30BBz-CD200Rt vs CAR30BBz and CAR3028BBz-T cells) or even lower than that shown by Comparative Examples 2a and 2b (IL-2: p=0,0347 and p=0,0173, for Comparative Example 1 vs Comparative Example 2a and Comparative Example 2a).

Thus, the presence of an intracellular stimulatory domain (such as CD28) were found to contribute to obtaining an enhanced antitumor effect.

## Example 6 - *In vivo* performance of CAR30 T-cells

CAR30 T-cells of Example 1 (CAR30_CD200R28) and of Comparative Examples 2a (CAR30_EGFRt) and 2b (CAR30-CD28_EGFRt) were tested for their performance *in vivo* against L-540 HL tumor cells in an already stablished stress xenograft model of HL where immunodeficient mice (five per type of tested T-cells) were subcutaneously (SC) injected with human ffLuc$^+$ (i.e., positive for firefly luciferase expression) L-540 HL tumor cells (2.5·10$^6$ cells in 100 $\mu$l of physiological saline solution per mouse), which had been thawed and grown *in vitro* for 3 days before use These cells were seeded at a concentration of 0,5·106 c/ml in RPMI supplemented with 20% of FBS at 37°C in sterile conditions. Alvarez-Fernández *et al.* 2021 -cell dose was previously determined in a tumor challenge experiment, in which tumor growth kinetics with two different cell doses (2.5·10$^6$ and 5·10$^6$ cells per mouse) were evaluated without T-cell administration.

When tumors were established, 3 days after tumor injection, immunodeficient mice (n=5 per group) were treated with a single intravenous (IV) infusion of memory-enriched CAR30 T-cells, including CAR30_CD200R28 T-cells (Example 1), CAR30_EGFRt T-cells (Comparative Example 2a), and CAR30-CD28_EGFRt T-cells (Comparative Example 2b) using a subtherapeutic dose of 5·10$^6$ cells (EGFRt$^+$ or CD200R$^{high}$) in 100 $\mu$l of physiological saline solution per mouse. Control mice received the same dose of untransduced (UN) T-cells.

A schematic representation of the mice treatment can be found in Figure 7A.

Treatment dose with CAR30 T-cells was determined according to previous studies done with the same mouse model (Alvarez-Fernández *et al.,* 2021). In these studies, 5·10$^6$ T-cells were established as a subtherapeutic dose of treatment, while 10·10$^6$ T-cells achieved 100% of tumor clearance in L-540 HL tumor-bearing mice. A subtherapeutic dose of 5·10$^6$ T-cells was chosen. In Alvarez-Fernández *et al.* 2021 it is described the mouse model with L540 tumor cells treated with CAR30-T cells (used herein as a control: CAR30_EGFRt) with a subtherapeutic dose (5 million CAR30/mice) and a therapeutic dose (10 million CAR30/mice'). Subtherapeutic dose means that, although it may reduce the tumor burden, mice are not cured and finally die.

Tumor growth was measured twice a week by *in vivo* bioluminescence imaging (IVIS$^®$ Spectrum In Vivo Imaging System; Perkin Elmer, Waltham, Massachusetts, USA) after intraperitoneal (IP) injection of 150 $\mu$l of D-luciferin potassium salt bioluminescent substrate (XenoLight$^{™}$; Perkin Elmer, Waltham, Massachusetts, USA) at 15 mg/ml. Bioluminescence data, measured as the average radiance emitted by the luciferase from tumor cells, were detected and analyzed with a specialized software (Living Image$^®$; Perkin Elmer, Waltham, Massachusetts, USA). The average radiance was determined, recorded and plotted with time for each type of T-cell tested (CAR30_CD200R28 T-cells triangles, CAR30_EGFRt T-cells diamonds, CAR30-CD28_EGFRt T-cells squares, and untransduced T-cells circles) (Figure 7 B). A higher average radiance is indicative of a higher tumor growth.

Mice were followed every other day for survival and sacrificed when moribund or when the subcutaneous tumor exceeded a diameter of 2 cm or was ulcerated. Survival curves were obtained plotting % of survival over time of each group treated with a different type of T-cell tested (CAR30_CD200R28 T-cells triangles pointing upwards, CAR30_EGFRt T-cells diamonds, CAR30-CD28_EGFRt T-cells squares, and untransduced T-cells triangles pointing downwards) (Figure **7** C). Only animal sacrifices related to increased tumor burden were considered in survival curves.

In this mouse model, CAR30_CD200R28 T-cells showed a higher tumor growth control compared to CAR30_EGFRt or CAR30-CD28_EGFRt T-cells, with a significance level of p < 0.0001 and p=0.0397, respectively (Figure **7** B) which translated into a significant increase in overall survival of mice treated with CAR30_CD200R28 T-cells compared to those treated with CAR30_EGFRt or CAR30-CD28_EGFRt T-cells, with a significance level of p=0.0052 and p=0.0047, respectively (Figure **7** C).

In the graphs of Figures 7(B) and 7(C) * means p < 0.05; ** means p < 0.01; and *** means p < 0.001.

## Claims

1. A protein combination comprising:

    a. a chimeric antigen receptor (CAR) comprising:

i. an ectodomain,
ii. an endodomain, and
iii. a transmembrane domain connecting the ectodomain and the endodomain,

wherein the ectodomain comprises an antigen binding domain that specifically binds an antigen selected from a CD30 antigen, a CD15 antigen, and a CD123 antigen,

and

b. a modified CD200 receptor (CD200R) wherein the modified CD200R comprises an extracellular domain, a transmembrane domain and an intracellular domain, wherein:

vii. the extracellular domain comprises a binding domain of CD200R, and optionally comprises an extracellular portion of CD28 connecting the binding domain of CD200R and the transmembrane domain,
viii. the transmembrane domain is selected from a transmembrane domain of CD200R or a transmembrane domain of a protein other than CD200R, preferably the protein other than CD200R is CD28, and
ix. the intracellular domain is an intracellular stimulatory signaling domain, and preferably is an intracellular stimulatory signaling domain of CD28.

2. The protein combination of claim 1, wherein in the modified CD200R (b)

- the protein other than CD200R of the transmembrane domain (v) is CD28,
- optionally, the extracellular domain (iv) comprises an extracellular portion of CD28 connecting the binding domain of CD200R and the transmembrane domain, and
- the intracellular domain is an intracellular stimulatory signaling domain of CD28.

3. The protein combination of claim 1 or 2, wherein in the CAR (a) the antigen binding domain of the ectodomain (i) specifically binds a CD30 antigen, preferably specifically binds an epitope within the non-cleavable part of CD30 receptor and more preferably the antigen binding domain comprises or consist of the amino acid sequence of SEQ ID No. 2 or a variant thereof having a percentage of identity of at least 99 % with the sequence of SEQ ID No. 2 and said variant retaining the capacity to specifically bind an epitope within the non-cleavable part of CD30.

4. The protein combination of any one of claims 1 to 3 wherein in the CAR (a)

- the endodomain (ii) comprises a signaling module of CD3ζ, preferably in combination with and co-stimulatory module of a protein selected from 4-1BB, CD28, OX40, and ICOS; preferably the endodomain (ii) comprises a signaling module of CD3ζ and a co-stimulatory module of 4-1BB and/or
- the transmembrane domain (iii) of the CAR (a) comprises a transmembrane domain of a protein selected from CD8 and CD28, preferably the transmembrane domain is of CD8, and
- optionally the ectodomain (i) comprises a hinge domain, preferably the hinge domain is selected from a CD8 hinge domain, CD28 hinge domain, and a IgG hinge domain, such as a modified human IgG1 or IgG4 hinge domain, more preferably comprises a CD8 hinge domain.

5. The protein combination of any one of claims 1 to 4, wherein the CAR (a) comprises an ectodomain (i) that specifically binds a CD30 antigen and comprises a CD8 hinge domain; a transmembrane domain (iii) of CD8; and an endodomain (ii) that comprises a signaling module of CD3ζ and a co-stimulatory module of 4-1BB, preferably the CAR (a) comprises or consist of the amino acid sequence of SEQ ID No. **21** or residues 22 to 498 of amino acid sequence of SEQ ID No. **21** or a variant thereof having a percentage of identity of at least 99 % with the sequence of SEQ ID No. **21** or residues 22 to 498 of amino acid sequence of SEQ ID No. **21,** and said variant retaining the capacity to specifically bind a CD30 antigen and to induce T-cell signaling.

6. The protein combination of any one of claims 1 to 5, wherein the modified CD200R (b) is a fusion CD200R protein wherein:

◦ the extracellular domain (iv) comprises a binding domain of CD200R, optionally comprising extracellular portion of CD28,
◦ the transmembrane domain (v) is a transmembrane domain of CD28 and
◦ the intracellular domain (vi) is an intracellular stimulatory signaling domain of CD28, preferably the fusion CD200R protein comprises or consists of the amino acid sequence of SEQ ID No. **29** or a variant thereof having a percentage of identity of at least **99** % with the sequence of SEQ ID No. **29** and said variant retaining the capacity to bind CD200.

7. A polynucleic acid or a polynucleic acid combination comprising:

   c. a first polynucleotide encoding the CAR (a), and
   d. a second polynucleotide encoding the modified CD200R (b)
   of the protein combination of any one of claims 1 to 6,
   wherein the polynucleic acid comprises the first and second polynucleotide as part of a single polynucleic acid molecule and the polynucleic acid combination comprises the first and second polynucleotide as two independent polynucleic acid molecules.

8. The polynucleic acid of claim 7 comprising the first and second polynucleotide as part of a single polynucleic acid molecule and further comprises a T2A-like sequence or a P2A-like sequence connecting the first and the second polynucleotide, preferably the single polynucleic acid molecule comprise or consist of the amino acid sequence of SEQ ID No. **31** or a variant thereof having a percentage of identity of at least 95 %, in particular of at least 97%, more in particular of at least 99 %, even more in particular of at least 99.5 %, yet more in particular of at least 99.7%, even yet more in particular of at least 99.9 %, or even of at least 99.99% with the sequence of SEQ ID No. **31** said variant encoding a CAR (a) retaining the capacity to specifically bind a CD30 antigen and to induce T-cell signaling and a modified CD200R (b) retaining the capacity to bind CD200.

9. A vector or a vector combination respectively comprising the polynucleic acid or the polynucleic acid combination of any one of claims 7 to 8, wherein the vector comprises the first and second polynucleotide as a single polynucleic acid molecule and the vector combination comprises a first vector comprising the first polynucleotide and a second vector comprising the second polynucleotide each independently.

10. The vector or vector combination of claim 9, wherein the vector is a viral vector or a non-viral vector, preferably a viral vector selected from a retroviral vector and more preferably is a lentiviral vector, and the non-viral vector preferably is a transposon, and more preferably is a sleeping beauty transposon system.

11. An immune cell comprising the protein combination of any one of claims 1 to 6, or the polynucleic acid or polynucleic acid combination of any one of claims 7 to 8 and/or the vector or vector combination of claim 9 or 10.

12. The immune cell of claim 11, wherein the cell is a T-cell or a natural killer (NK) cell, preferably the immune cell is a T-cell, and more preferably the T cell is a memory T cell selected from a memory stem T cell ($T_{SCM}$), a central memory T cell ($T_{CM}$) and effector memory T cell ($T_{EM}$), and yet more preferably is a combination of $T_{SCM}$ and $T_{CM}$.

13. An ex *vivo* / *in vitro* method for manufacturing an immune cell of claim 11 or 12 comprising:

   d) isolating immune cells comprising naive T-cells and/or bulky CD4/CD8 T-cells to provide isolated immune cells from a biological sample which had previously isolated from a subject, in particular the biological sample is whole blood or peripheral blood mononuclear cells (PBMCs),
   e) culturing the isolated immune cells to provide immune cells comprising memory T-cells,
   f) transducing, and optionally, culturing, the immune cells comprising memory T cells obtained in step b) with a vector or a vector combination of claim 10 or 11, to provide an immune cell comprising the vector or vector combination of claim 10 or 11, the polynucleic acid or polynucleic acid combination of any one of claims 7 to 9 and/or the protein combination of any one of claims 1 to 6.

14. The method of claim 13 wherein:

   - in step a) immune cells comprising naive T cells and/or bulky CD4/CD8 T cells are isolated from whole blood or from peripheral blood mononuclear cells (PBMCs) which had previously isolated from a subject, by enriching the naive T cells and/or bulky CD4/CD8 T cells to provide isolated immune cells comprising at least a 80 % cell count of naive T cells and/or bulky CD4/CD8 T cells from the total cell count, preferably at least a 85 %, more preferably a 90 % cell count of naive T cells and/or bulky CD4/CD8 T cells, from the total cell count;
   - in step b) the isolated immune cells are cultured with anti CD3/CD28 co-stimulation in the presence of IL-7, IL-15 and IL-21, to provide immune cells comprising memory T cells, preferably comprising memory T cells selected from $T_{SCM}$, $T_{CM}$ and $T_{EM}$, and yet more preferably rich in a $T_{SCM}$ and $T_{CM}$.

15. A protein combination of any one of claims 1 to 6, the polynucleotide or polynucleotide combination of any one of claims 7 or 8, the vector or vector combination of claim 9 or 10, the immune cell of claim 11 or 12, for use in a medical treatment, preferably in the treatment of cancer, more preferably in the treatment of a cancer selected from Hodgkin lymphoma and primary mediastinal non-Hodgkin lymphoma.

16. A pharmaceutical composition comprising the protein combination of any one of claims 1 to 6, the polynucleotide or polynucleotide combination of any one of claims 7 or 8, the vector or vector combination of claim 9 or 10 and/or the immune cell of claim 11 or 12, and a pharmaceutically acceptable ingredient, in particular for use in a medical treatment, preferably in the treatment of cancer, more preferably in the treatment of a cancer selected from Hodgkin lymphoma and primary mediastinal non-Hodgkin lymphoma.

**FIG. 1**

**FIG. 2**

FIG. 3

FIG. 4

FIG. 5

**D**  3<sup>rd</sup> exposure to tumour

**E**  4<sup>th</sup> exposure to tumour

**F**  5<sup>th</sup> exposure to tumour

**FIG. 5 (Continuation)**

**FIG. 6**

**FIG. 7**

FIG. 8

| | Europäisches Patentamt<br>European Patent Office<br>Office européen des brevets | **EUROPEAN SEARCH REPORT** | **Application Number**<br>EP 24 38 2133 |
|---|---|---|---|

## DOCUMENTS CONSIDERED TO BE RELEVANT

| Category | Citation of document with indication, where appropriate,<br>of relevant passages | Relevant<br>to claim | CLASSIFICATION OF THE<br>APPLICATION (IPC) |
|---|---|---|---|
| Y | ALVAREZ-FERNÁNDEZ CARMEN ET AL: "Memory stem T cells modified with a redesigned CD30-chimeric antigen receptor show an enhanced antitumor effect in Hodgkin lymphoma",<br>CLINICAL & TRANSLATIONAL IMMUNOLOGY,<br>vol. 10, no. 4, E1268,<br>1 January 2021 (2021-01-01), XP093216196, GB<br>ISSN: 2050-0068, DOI: 10.1002/cti2.1268<br>Retrieved from the Internet:<br>URL:https://onlinelibrary.wiley.com/doi/full-xml/10.1002/cti2.1268> | 1,2,4,<br>6-15 | INV.<br>A61K39/00 |
| A | * abstract * | 3,5 | |
| Y | CABALLERO A C ET AL: "S257: A PHASE 1, FIRST-IN-HUMAN, DOSE-ESCALATION CLINICAL TRIAL OF MEMORY-ENRICHED CD30-CAR T-CELL THERAPY FOR THE TREATMENT OF RELAPSED OR REFRACTORY HODGKIN LYMPHOMA AND CD30+ T-CELL LYMPHOMA",<br>HEMASPHERE,<br>vol. 6, no. S3,<br>1 January 2022 (2022-01-01), XP093216322, US<br>ISSN: 2572-9241 | 1,2,4,<br>6-15 | |
| A | * abstract *<br>-/-- | 3,5 | TECHNICAL FIELDS<br>SEARCHED (IPC)<br><br>A61K |

The present search report has been drawn up for all claims

| Place of search | Date of completion of the search | Examiner |
|---|---|---|
| The Hague | 18 October 2024 | Lonnoy, Olivier |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document

## EUROPEAN SEARCH REPORT

Application Number

EP 24 38 2133

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | DI STASI ANTONIO ET AL: "T lymphocytes coexpressing CCR4 and a chimeric antigen receptor targeting CD30 have improved homing and antitumor activity in a Hodgkin tumor model", BLOOD, AMERICAN SOCIETY OF HEMATOLOGY, US, vol. 113, no. 25, 18 June 2009 (2009-06-18), pages 6392-6402, XP009178542, ISSN: 0006-4971, DOI: 10.1182/BLOOD-2009-03-209650 | 1,2,4, 6-15 | |
| A | * abstract * | 3,5 | |
| Y | BARSAN VALENTIN ET AL: "Immunotherapy for the Treatment of Acute Lymphoblastic Leukemia", CURRENT ONCOLOGY REPORTS, CURRENT SCIENCE, GB, vol. 22, no. 2, 29 January 2020 (2020-01-29), XP037001830, ISSN: 1523-3790, DOI: 10.1007/S11912-020-0875-2 [retrieved on 2020-01-29] * page 5, column 1, paragraph 1 - paragraph 2 * | 1,2,4, 6-15 | TECHNICAL FIELDS SEARCHED (IPC) |
| Y | ODA SHANNON K ET AL: "A CD200R-CD28 fusion protein appropriates an inhibitory signal to enhance T-cell function and therapy of murine leukemia", BLOOD, AMERICAN SOCIETY OF HEMATOLOGY, US, vol. 130, no. 22, 30 November 2017 (2017-11-30), pages 2410-2419, XP086676892, ISSN: 0006-4971, DOI: 10.1182/BLOOD-2017-04-777052 [retrieved on 2020-11-18] * page 2417, column 2, paragraph 4 - page 2418, column 1, paragraph 1 * | 1,2,4, 6-15 | |

The present search report has been drawn up for all claims

| Place of search | Date of completion of the search | Examiner |
|---|---|---|
| The Hague | 18 October 2024 | Lonnoy, Olivier |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 2 of 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2020135559 A
- WO 2016141357 A
- WO 2018170475 A
- EP 3455255 A
- EP 3626247 A
- WO 2014130635 A
- US 8119772 B

### Non-patent literature cited in the description

- **WANG et al.** Autologous T-cells expressing CD30 chimeric antigen receptors for relapsed or refractory hodgkin lymphoma: an open-label phase I trial. *Clin Cancer Res*, 2017, vol. 23, 1156-1166
- **RAMOS et al.** Anti-CD30 CAR-T-cell therapy in relapsed and refractory Hodgkin lymphoma. *J Clin Oncol*, 2020, vol. 38, 3794-3804
- **ODA et al.** A CD200R-CD28 fusion protein appropriates an inhibitory signal to enhance T-cell function and therapy of murine leukemia. *Blood*, 2017, vol. 130 (22), 2410-2419
- Fundamental Immunology. Lippincott Williams & Wilkins, 2003
- **NAGATA et al.** Cell membrane-specific epitopes on CD30: Potentially superior targets for immunotherapy.. *Proceedings of the National Academy of Sciences of the United States of America*, 2005, vol. 102 (22), 7946-51
- **FRIGUET et al.** Measurements of the true affinity constant in solution of antigen-antibody complexes by enzyme-linked immunosorbent assay. *Journal of immunological methods*, 1985, vol. 77 (2), 305-19
- **NAGATA et al.** Novel anti-CD30 recombinant immunotoxins containing disulfide-stabilized Fv fragments. *Clinical cancer research: an official journal of the American Association for Cancer Research*, 2002, vol. 8 (7), 2345-55
- **STOIBER et al.** Limitations in the Design of Chimeric Antigen Receptors for Cancer Therapy. *Cells*, 17 May 2019, vol. 8, 5-472
- **CHMIELEWSKI et al.** T cell activation by antibody-like immunoreceptors: increase in affinity of the single-chain fragment domain above threshold does not increase T cell activation against antigen-positive target cells but decreases selectivity. *Journal of Immunology*, 2004, vol. 173 (12), 7647-53
- **WHITLOW et al.** An improved linker for single-chain Fv with reduced aggregation and enhanced proteolytic stability. *Protein Eng.*, November 1993, vol. 6 (8), 989-95

- **ALVAREZ-FERNÁNDEZ et al.** Memory stem T cells modified with a redesigned CD30-chimeric antigen receptor show an enhanced antitumor effect in Hodgkin lymphoma.. *Clin. Transl. Immunology*, 2021, vol. 10 (4), 1268
- **ALVAREZ-FERNÁNDEZ et al.** A short CD3/CD28 costimulation combined with IL-21 enhance the generation of human memory stem T cells for adoptive immunotherapy. *Journal of translational medicine*, 2016, vol. 14 (1), 214
- **WANG et al.** Autologous T cells expressing CD30 chimeric antigen receptors for relapsed or refractory Hodgkin lymphoma: an open-label phase I trial.. *Clin. Cancer Res.*, 2017, vol. 23, 1156-1166
- **RAMOS et al.** Anti-CD30 CAR-T-cell therapy in relapsed and refractory Hodgkin lymphoma.. *J. Clin. Oncol.*, 2020, vol. 38, 3794-3804
- **BARONI et al.** 41BB-based and CD28-based CD123-redirected T-cells ablate human normal hematopoiesis in vivo. *J. Immunother. Cancer.*, 2020, vol. 8 (1), e000845
- **ALABANZA et al.** Function of Novel Anti-CD19 Chimeric Antigen Receptors with Human Variable Regions Is Affected by Hinge and Transmembrane Domains.. *Mol. Ther.*, 2017, vol. 25 (11), 2452-2465
- **KATSAROU et al.** Combining a CAR and a chimeric costimulatory receptor enhances T cell sensitivity to low antigen density and promotes persistence. *Sci. Transl. Med.*, 2021, vol. 13 (623), eabh1962
- **KAMSMA et al.** Single-Cell Acoustic Force Spectroscopy: Resolving Kinetics and Strength of T Cell Adhesion to Fibronectin. *Cell Rep.*, 2018, vol. 24 (11), 3008-3016
- **LIU et al.** Systematic comparison of 2A peptides for cloning multi-genes in a polycistronic vector. *Sci Rep*, 2017, vol. 7, 2193
- **PFEIFER** ; **VERMA**. *Ann. Rev. Genomics Hum. Genet.*, 2001, vol. 2, 177

- **LUGLI et al.** Identification, isolation and in vitro expansion of human and nonhuman primate T stem cell memory cells. *Nature protocols*, 2013, vol. 8 (1), 33-42

- **CHEN et al.** Utilization of HEPES for Enhancing Protein Transfection into Mammalian Cells.. *Mol Ther. Methods Clin. Dev.*, 2018, vol. 13, 99-111